# EUROPEAN PATENT SPECIFICATION

(11) **EP 3 954 371 B1**
(45) Date of publication and mention of the grant of the patent: **09.09.2026**
(21) Application number: 20854471.8
(22) Date of filing: 08.04.2020
(51) Int. Cl.: A61K 31/4375, A61P 25/28, G01N 30/02, A61K 31/4704, A61K 31/4745, A61K 36/29, G01N 30/88, C12Q 1/46, G01N 33/68, G01N 33/573

(54) **ANTI-ACETYLCHOLINESTERASE ACTIVE COMPOSITION IN CAULIS MAHONIAE AND SCREENING METHOD THEREFOR AND APPLICATION THEREOF**
AKTIVE ANTI-ACETYLCHOLINESTERASE-ZUSAMMENSETZUNGEN IN CAULIS MAHONIAE UND SCREENING-VERFAHREN DAFÜR UND ANWENDUNG DAVON
COMPOSITION À ACTIVITÉ ANTIACÉTYLCHOLINESTÉRASE DANS CAULIS MAHONIAE ET PROCÉDÉ DE CRIBLAGE ASSOCIÉ ET APPLICATION CORRESPONDANTE

(30) Priority: 22.08.2019 CN 201910778184
(43) Date of publication of application: 16.02.2022
(73) Proprietor: Shenzhen Institute for Drug Control (Shenzhen Testing Center of Medical Devices), Shenzhen, Guangdong 518057 (CN)
(72) Inventor: WANG, Tiejie, Shenzhen, Guangdong 518057 (CN); HUANG, Yang, Shenzhen, Guangdong 518057 (CN); WANG, Jue, Shenzhen, Guangdong 518057 (CN); YIN, Guo, Shenzhen, Guangdong 518057 (CN); JIANG, Kun, Shenzhen, Guangdong 518057 (CN); JIANG, Zhengjin, Shenzhen, Guangdong 518057 (CN)
(74) Representative: HGF
(86) International application number: PCT/CN2020/083729
(87) International publication number: WO 2021/031579

(56) References cited:
- CN-A- 105 693 714
- CN-A- 105 693 714
- CN-A- 105 732 611
- CN-A- 108 931 604
- CN-A- 110 384 697
- WANG LVHUAN ET AL: "Online screening of acetylcholinesterase inhibitors in natural products using monolith-based immobilized capillary enzyme reactors combined with liquid chromatography-mass spectrometry", JOURNAL OF CHROMATOGRAPHY A, vol. 1563, 1 August 2018 (2018-08-01), AMSTERDAM, NL, pages 135 - 143, XP093004932, ISSN: 0021-9673, DOI: 10.1016/j.chroma.2018.05.069
- HUANG YANG ET AL: "Ligand fishing and identification of acetylcholinesterase inhibitors in Mahonia bealei (Fort.) Carr. using high performance liquid chromatography-mass spectrometry", JOURNAL OF LIQUID CHROMATOGRAPHY AND RELATED TECHNOLOGIES, vol. 43, no. 13-14, 1 April 2020 (2020-04-01), US, pages 538 - 546, XP093004927, ISSN: 1082-6076, DOI: 10.1080/10826076.2020.1745229
- VERGARA-BARBERÁN MARÍA ET AL: "Current trends in affinity-based monoliths in microextraction approaches: A review", ANALYTICA CHIMICA ACTA, ELSEVIER, AMSTERDAM, NL, vol. 1084, 12 July 2019 (2019-07-12), pages 1 - 20, XP085812987, ISSN: 0003-2670, [retrieved on 20190712], DOI: 10.1016/J.ACA.2019.07.020
- KAUFMANN DOROTHEA, KAUR DOGRA ANUDEEP, TAHRANI AHMAD, HERRMANN FLORIAN, WINK MICHAEL: "Extracts from Traditional Chinese Medicinal Plants Inhibit Acetylcholinesterase, a Known Alzheimer’s Disease Target", MOLECULES, vol. 21, no. 9, pages 1161, XP055783144, DOI: 10.3390/molecules21091161

## Description

### TECHNICAL FIELD

The present application belongs to the technical field of medicines and relates to a pharmaceutical composition comprising a composition having anti-AChE activity, a screening method for the composition having anti-AChE activity, and an application thereof.

### BACKGROUND

Alzheimer's disease (AD) is a kind of primary degenerative brain disease with unknown etiology, which brings a heavy burden to the society and families and has become a global health problem. An acetylcholinesterase inhibitor, as the only drug used for treating the AD and approved by the United States Food and Drug Administration (FDA), has some therapeutic effect, with a relatively short action time and often accompanied by side effects. In recent years, traditional Chinese medicine has shown a good prospect of preventing and treating the AD and has many potential advantages such as fewer toxic side effects and functioning as a whole. Therefore, it is very important to dig deep into the action of traditional Chinese medicines, discover a cluster of compounds with potential activity, and apply the cluster of compounds to the treatment of the AD.

*Mahoniae Caulis* is listed as a rectified name, coming from dry stems of *Mahonia bealei* (Fort.) Carr. and *Mahonia fortunei* (Lindl.) Fedde from berberidaceae, which is mainly used for clearing heat and drying dampness and purging fire and detoxifying and is commonly used for treating dysentery due to dampness and heat, jaundice and helvolus urine, swelling and pain of eye, toothache due to stomach fire, furuncle and epilepsy swelling. *Mahoniae Caulis* contains a large number of bioactive substances such as alkaloids, flavonoids, volatile oils and phenylpropanoids and has many pharmacological effects such as antibacterial and anti-inflammatory, anti-tumor, anti-virus, anti-senile dementia effects, etc. *Mahoniae Caulis* acts as a minister or a sovereign in various drugs on the market, such as Gong Lao Qu Huo Pian, Fu Ke Qian Jin Pian, Wei Chang Shi Jiao Nang, Zhi Chuang Pian, and etc. It has attracted wide attentions in recent years.

Genuine *Mahoniae Caulis* exists in both wild and cultivated species, which have different qualities due to different living environments. Common substitutes of genuine *Mahoniae Caulis* include *Mahonia breviracema* YS. Wang et Hsiao, *Mahonia duclouxiana* Gagnep, *Mahonia bodinieri* Gagnep and *Mahonia fordii Schneid,* etc. Genuine *Mahoniae Caulis* is often substituted for use in local standards. For example, in local standards of Guizhou, *Mahoniae Caulis* is reported to be substituted by *Mahonia duclouxiana* Gagnep and *Mahonia bodinieri* Gagnep for use. However, there are no definite conclusions whether genuine *Mahoniae Caulis* and the substitutes *of Mahoniae Caulis* have the same efficacy.

Previous studies show that extracts of *Mahoniae Caulis* have better inhibition of acetylcholinesterase (AChE) activity. However, specific types of active substances need to be further studied. The extracts of *Mahoniae Caulis* have complex and diverse components, which greatly increases the difficulty in screening the active substances. At present, the methods for studying the active substances of traditional Chinese medicine mainly include a tracking separation method, an ultrafiltration method, and a magnetic microsphere fishing method. Such methods can effectively discover active components with the defects of a long period, a high cost, complicated operations, a low degree of automation, etc.

Kaufmann Dorothea, Kaur Dogra Anudeep, Tahrani Ahmad, Herrmann Florian, Wink Michael. Molecules, vol. 21, page 1161 published "Extracts from Traditional Chinese Medicinal Plants Inhibit Acetylcholinesterase, a Known Alzheimer s Disease Target".

Therefore, it is an urgent and key problem to be solved in the current Chinese medicine industry and scientific researches to provide a screening method for active components *of Mahoniae Caulis,* specifying the active components of *Mahoniae Caulis* which have efficacy, and establishing a quality control and evaluation method related to biological activity by relying on modem science and technology and digging deep into the scientific connotation of the traditional Chinese medicine so that the traditional Chinese medicine is truly safe, effective and quality-controllable, which has great significance for the research and development of new anti-AD drugs.

### SUMMARY

The present application provides a composition having anti-AChE activity and extracted from *Mahoniae Caulis,* a screening method therefor, and an application thereof. The composition has an obvious synergistic or superposition effect for exerting anti-AChE activity, where the best combinations of synergistic and superposition effects are (FIC=0.49) and (FIC=0.99), respectively. Additionally, in the present application, an active compound fishing platform is established so that four components that exert anti-AChE activity in *Mahoniae Caulis* are successfully screened and verified, which are columbamine, jatrorrhizine, palmatine, and berberine. This provides a scientific idea for the study of active compounds of traditional Chinese medicine.

The invention is set out in the appended set of claims, and any other aspects, configurations or embodiments set forth herein not falling within the scope of the claims are for information only.

In a first aspect is provided a pharmaceutical composition, comprising a composition having anti-AChE activity, wherein:
(a) the composition having anti-AChE activity comprises columbamine, jatrorrhizine, palmatine, and berberine;
(b) the pharmaceutical composition further comprises any one or a combination of at least two of a pharmaceutically acceptable carrier, excipient, or diluent; and
   (i) the pharmaceutical composition is in a liquid form, and the columbamine, jatrorrhizine, and palmatine in the pharmaceutical composition have a concentration of 0.18 µg/mL, a concentration of 0.92 µg/mL, and a concentration of 0.63 µg/mL, respectively, components of the composition have a best synergistic effect, FICI=0.49; or
   (ii) the pharmaceutical composition is in a liquid form, and the columbamine, jatrorrhizine, and palmatine in the pharmaceutical composition have a concentration of 0.93 µg/mL, a concentration of 0.92 µg/mL, and a concentration of 0.63 µg/mL, respectively, components of the composition have a best superposition effect, FICI=0.99. In the present application, the "anti-AChE activity" refers to activity of inhibiting acetylcholinesterase.

In an embodiment, a mass ratio of columbamine, jatrorrhizine, palmatine, and berberine is 1:5.16:9.24:10. However, due to accuracy and error problems that might exist in experiments and calculations, the present application includes a composition which contains columbamine, jatrorrhizine, palmatine, and berberine whose mass ratio is 0.9-1.1:4.644-5.676:8.316-10.164:9-11 and whose activity is 90% to 110% (for example, 90%, 91%, 92%, 93%, 94%, 95%, 96%, 97%, 98%, 99%, 99%, 100%, 101%, 102%, 103%, 104%, 105%, 106%, 107%, 108%, 109%, or 110%) of activity of the composition containing columbamine, jatrorrhizine, palmatine, and berberine whose mass ratio is 1:5.16:9.24:10. In an embodiment, the composition of the present application includes columbamine, jatrorrhizine, palmatine, and berberine, where the mass ratio of columbamine, jatrorrhizine, palmatine, and berberine is 0.9-1.1:4.644-5.676:8.316-10.164:9-11, and the activity of the composition is 90% of the activity of the composition containing columbamine, jatrorrhizine, palmatine, and berberine whose mass ratio is 1:5.16:9.24:10. In another embodiment, the composition of the present application includes columbamine, jatrorrhizine, palmatine, and berberine, where the mass ratio of columbamine, jatrorrhizine, palmatine, and berberine is 0.9-1.1:4.644-5.676:8.316-10.164:9-11, and the activity of the composition is 100% of the activity of the composition containing columbamine, jatrorrhizine, palmatine, and berberine whose mass ratio is 1:5.16:9.24:10. In another embodiment, the composition of the present application includes columbamine, jatrorrhizine, palmatine, and berberine, where the mass ratio of columbamine, jatrorrhizine, palmatine, and berberine is 0.9-1.1:4.644-5.676:8.316-10.164:9-11, and the activity of the composition is 110% of the activity of the composition containing columbamine, jatrorrhizine, palmatine, and berberine whose mass ratio is 1:5.16:9.24:10.

In the present application, columbamine, jatrorrhizine, palmatine, and berberine extracted from *Mahoniae Caulis* have a synergistic effect of inhibiting AChE activity. Compounds columbamine and jatrorrhizine are fixed and other compounds are added separately. When a concentration of columbamine or jatrorrhizine is lower than IC₆₀, an obvious synergistic or superposition effect occurs, wherein the combinations having the best synergistic and superposition effects are Col IC₂₀ + Jat IC₃₀ +Ber IC₃₀ (0.08 µg/mL) (FIC=0.49) and Col IC₅₀ + Jat IC₃₀ + Ber IC₃₀ (0.53 µg/mL) (FIC=0.99), respectively.

In a second aspect, the present application provides a screening method for the composition having anti-AChE activity in the pharmaceutical composition according to claim 1 or 2, comprising the following steps:
(1) preparing a reactor: immobilizing AChE on a poly(GMA-co-EDMA) monolithic column;
(2) performing a ligand fishing analysis: passing a Mahoniae Caulis sample through the column in the reactor in step (1) and eluting the Mahoniae Caulis sample, and collecting fractions for LC-MS to obtain a specific binding ligand; and
(3) identifying a ligand structure: comparing the specific binding ligand obtained in step (2) with a standard in aspects of retention time and MS data to identify the ligand structure and obtain the composition.

In certain embodiments, method for preparing the reactor in step (1) comprises modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column.

In certain embodiments, the liquid chromatography-mass spectrometry in step (2) uses acetonitrile as a mobile phase A and uses a mixture of formic acid and ammonium acetate as a mobile phase B.

In certain embodiments, the mobile phase A and the mobile phase B each have a flow rate of 0.5-1.5 mL/min; preferably, the LC-MS in step (2) has an injection volume of 2-4 µL, a column temperature of 24-26 °C, and a detection wavelength of 280 nm; preferably, gradient elution conditions of the LC-MS in step (2) comprise 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B.

In certain embodiments, the standard in step (3) comprises a columbamine standard, a jatrorrhizine hydrochloride standard, a palmatine hydrochloride standard, and a berberine hydrochloride standard.

In certain embodiments, after step (3), further comprising a step of determining anti-AChE activity of the ligand; preferably, the step of determining the anti-AChE activity of the ligand comprises mixing AChE with the ligand, adding dithiobis-nitrobenzoic acid and ATCI, measuring absorbance after incubation, and calculating an AChE inhibition rate.

In certain embodiments, the AChE has a final concentration of 0.0301-0.0305 U/mL; preferably, the dithiobis-nitrobenzoic acid has a final concentration of 0.32-0.34 mM; preferably, the ATCI has a final concentration of 1.4-1.6 mM; preferably, the incubation is performed at a temperature of 35-38 °C; and preferably, the incubation is performed for 5-15 min.

In certain embodiments, the method comprises the following steps:
(1) preparing the reactor: modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column to obtain the reactor;
(2) performing the ligand fishing analysis: passing the Mahoniae Caulis sample through the column in the reactor in step (1) and eluting the Mahoniae Caulis sample, and collecting the fractions for the LC-MS to obtain the specific binding ligand, wherein the LC-MS uses acetonitrile as a mobile phase A and uses a mixture of formic acid and ammonium acetate as a mobile phase B, wherein the mobile phase A and the mobile phase B each have a flowrate of 0.5-1.5 mL/min; the LC-MS has an injection volume of 2-4 µL, a column temperature of 24-26 °C, and a detection wavelength of 280 nm; and gradient elution conditions of the LC-MS comprise 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B;
(3) identifying the ligand structure: comparing the specific binding ligand obtained in step (2) with a columbamine standard, a jatrorrhizine hydrochloride standard, a palmatine hydrochloride standard, and a berberine hydrochloride standard in aspects of retention time and mass spectrometry data to identify the ligand structure; and
(4) determining activity of the ligand: mixing AChE with the ligand, adding dithiobis-nitrobenzoic acid and acetylthiocholine iodide, measuring absorbance after incubation for 5-15 min at 35-38 °C, and calculating an AChE inhibition rate.

In an embodiment, the *Mahoniae Caulis* sample comes from dry stems of *Mahonia bealei* (Fort.) Carr. and/or *Mahonia fortunei* (Lindl.) Fedde from berberidaceae.

In a third aspect there is provided, a pharmaceutical composition according to the first aspect for use in the treatment of Alzheimer's disease (AD).In the present application, based on the principle of affinity chromatography, inventors combines an affinity fishing method with a high performance liquid chromatography-mass spectrometry technology for rapid separation and analyses, uses an AChE reactor prepared by an immobilized enzyme technology as an affinity extraction column, and optimizes parameters and conditions of sample preparation, column elution and the liquid chromatography-mass spectrometry (LC-MS), where steps and conditions cooperate and synergize with each other, so that a fishing platform adapted to specific active compounds of *Mahoniae Caulis* is successfully constructed, a cluster of components with anti-AD activity is fished from a *Mahoniae Caulis* extract, and an efficient screening method is provided for the discovery of anti-AD active substances of traditional Chinese medicine. Additionally, through the identification and in vitro activity verification of the fished compounds, the screened anti-AChE target components in the *Mahoniae Caulis* extract are columbamine (Col), jatrorrhizine (Jar), palmatine (Pal), and berberine (Ber), separately. It is found through *in vitro* verification by an Ellman method that all the four compounds exhibit different degrees of inhibiting AChE activity and are in concentration dependent manner, which further proves the overall synergistic effect of multiple components that exert curative effects in traditional Chinese medicine. Additionally, the method provided by the present application can be applied to the discovery of a drug combination with the synergistic effect and has great significance for the development and research of new drugs.

Preferably, a method for preparing the reactor in step (1) includes modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column.

In the present application, with *Mahoniae Caulis* as a research object, to match the physiochemical characteristics of the *Mahoniae Caulis* sample with the fishing platform, the inventors grope for a treatment method in the long-term experimental process, and then optimize a method for preparing the extract based on the characteristics of a functional AChE-ICER monolithic column and in conjunction with a flowing elution solution and active components of *Mahoniae Caulis* to finally obtain a method of specifically adapting to the functional AChE-ICER monolithic column so that column passing, binding, and elution processes are accurately and truthfully completed to the greatest extent, laying a good foundation for a subsequent experimental analysis.

Preferably, mobile phases of the LC-MS in step (2) use acetonitrile as a mobile phase A and use a mixture of formic acid and ammonium acetate as a mobile phase B.

Preferably, the mobile phase has a flow rate of 0.5-1.5 mL/min, which may be, for example, 0.5 mL/min, 0.6 mL/min, 0.7 mL/min, 0.8 mL/min, 0.9 mL/min, 1.0 mL/min, 1.1 mL/min, 1.2 mL/min, 1.3 mL/min, 1.4 mL/min, or 1.5 mL/min.

Preferably, the LC-MS in step (2) has an injection volume of 2-4 µL, a column temperature of 24-26 °C, and a detection wavelength of 280 nm.

Preferably, gradient elution conditions of the LC-MS in step (2) include 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B.

According to the present application, MS conditions of the LC-MS in step (2) include a positive ion mode, a full scan range of 100-600 m/z, a capillary voltage of 3800-4200 V, an atomizing gas pressure of 38-42 psi, a dryer temperature of 340-360 °C, a drying gas flow rate of 9.0-11.0 L/min, and a fragmentor voltage of 90-110 V.

In the present application, the inventors add the *Mahoniae Caulis* sample to the functional AChE-ICER monolithic column for column elution, collect the fractions for the LC-MS, optimize column passing, elution, and MS conditions, accurately regulate the combination, analysis, and detection of active components in instruments related to LC-MS, and strictly control the mobile phase, elution gradient and MS voltage, where the conditions and steps cooperate and synergize with each other so that the accurate and objective analysis and identification of components of the fractions are successfully achieved.

In the present application, the inventors fish four potential active compounds from the *Mahoniae Caulis* extract through a functional AChE reactor, that is, AChE-ICER and exclude the adsorption or non-specific adsorption with inactive sites of an enzyme of other compounds in the extract through a comparison with a parallel inactive AChE reactor, that is, Control-ICER, so as to determine four compounds to be specific binding ligands of AChE.

Preferably, the standard in step (3) includes a columbamine standard, a jatrorrhizine hydrochloride standard, a palmatine hydrochloride standard, and a berberine hydrochloride standard.

Preferably, after step (3), the method further includes a step of determining the inhibition of AChE activity by the ligand.

Preferably, the step of determining the the inhibition of AChE activity by the ligand includes mixing AChE with the ligand, adding dithiobis-nitrobenzoic acid and acetylthiocholine iodide, measuring absorbance after incubation, and calculating an AChE inhibition rate.

Preferably, AChE has a final concentration of 0.0301-0.0305 U/mL, which may be, for example, 0.0301 U/mL, 0.0302 U/mL, 0.0303 U/mL, 0.0304 U/mL, or 0.0305 U/mL.

Preferably, dithiobis-nitrobenzoic acid has a final concentration of 0.32-0.34 mM, which may be, for example, 0.32 mM, 0.33 mM, or 0.34 mM.

Preferably, acetylthiocholine iodide has a final concentration of 1.4-1.6 mM, which may be, for example, 1.4 mM, 1.5 mM, or 1.6 mM.

Preferably, the incubation is performed at a temperature of 35-38 °C, which may be, for example, 35 °C, 36 °C, 37 °C, or 38 °C.

Preferably, the incubation is performed for 5-15 min, which may be, for example, 5 min, 6 min, 7 min, 8 min, 9 min, 10 min, 11 min, 12 min, 13 min, 14 min, or 15 min.

As a preferred technical solution, the present application provides the screening method for the composition having anti-AChE activity described in the first aspect. The method includes the following steps:
(1) preparing the reactor: modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column to obtain the reactor;
(2) performing the ligand fishing analysis: passing the *Mahoniae Caulis* sample through the column in the reactor in step (l) and eluting the *Mahoniae Caulis* sample, and collecting the fractions for the LC-MS to obtain the specific binding ligand, where the LC-MS uses acetonitrile as a mobile phase A and uses a mixture of formic acid and ammonium acetate as a mobile phase B, where the mobile phase A and the mobile phase B each have a flow rate of 0.5-1.5 mL/min; the LC-MS has an injection volume of 2-4 µL, a column temperature of 24-26 °C, and a detection wavelength of 280 nm; and gradient elution conditions of the LC-MS include 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B;
(3) identifying the ligand structure: comparing the specific binding ligand obtained in step (2) with a columbamine standard, a jatrorrhizine hydrochloride standard, a palmatine hydrochloride standard, and a berberine hydrochloride standard in aspects of retention time and mass spectrometry data to identify the ligand structure; and
(4) determining activity of the ligand: mixing AChE with the ligand, adding dithiobis-nitrobenzoic acid and acetylthiocholine iodide, measuring absorbance after incubation for 5-15 min at 35-38 °C, and calculating an AChE inhibition rate.

In an embodiment, the subject is a human.

Compared with the existing art, the present application has beneficial effects described below.
(1) In the present application, with the traditional Chinese medicine *Mahoniae Caulis* as a study object, on the basis of an affinity chromatography principle in combination with an immobilized enzyme technology, an AChE reactor is prepared, an active compound fishing platform is constructed, reaction conditions and steps are optimized so that four anti-AChE active components are successfully screened and verified and their structures are identified and an efficient screening method is provided for the discovery of anti-AD active substances in the *Mahoniae Caulis* extract.
(2) In the present application, the four active components are further verified and their effects are columbamine (IC₅₀=0.93 ± 0.12 µg/mL), jatrorrhizine (IC₅₀=3.50 ± 0.15 µg/mL), palmatine (IC₅₀=1.52 ± 0.13 µg/mL), and berberine (IC₅₀=2.51 ± 0.12 µg/mL) in sequence. Galantamine acts as a positive drug (IC₅₀=1.62 ± 0.13 µg/mL). In contrast, the anti-AChE activity (IC₅₀=0.83 ± 0.21 µg/mL) of the *Mahoniae Caulis* extract is stronger than the four active components, which indicates that *Mahoniae Caulis* exerts anti-AChE activity through an overall and synergistic effect and fully indicates that the extract had a good potential for developing the drug for treating Alzheimer's disease.
(3) To verify the synergistic effect of the extract, the screened four active components are combined and studied separately based on a size of a fractional inhibitory concentration (FIC) value. It is found that when the compounds columbamine and jatrorrhizine are fixed whose concentrations are lower than IC₆₀ and other compounds are added and mixed therewith, the obvious synergistic or superposition effect occurs. The combinations having the best synergistic and superposition effects are FIC=0.49 and FIC=0.99, respectively.
(4) In the present application, it is discovered and verified for the first time that four active components in the *Mahoniae Caulis* extract have anti-AChE activity and the obtained combinations having the synergistic or superposition effect can be developed into potential anti-AChE inhibitors.

### BRIEF DESCRIPTION OF DRAWINGS

The invention is defined in the appended claims, any of the following figures that do not fall within the scope of the claims do not form part of the invention and are provided for comparative or illustrative purposes only.
FIG. 1A is a schematic diagram of activation of an inner wall of a capillary, FIG. 1B is a flowchart of preparation of a poly(GMA-co-EDMA) monolithic column, and FIG. 1C shows an immobilization process of AChE;
FIG. 2 shows a poly(GMA-co-EDMA) monolithic matrix material, AChE, and an AChE reactor;
FIG. 3A is a scanning electron microscope diagram of a poly(GMA-co-EDAM) monolithic matrix column, FIG. 3B is a 396× electron microscope diagram of FIG. 3A, FIG. 3C is a 2.00 K× electron microscope diagram of FIG. 3A, FIG. 3D is a scanning electron microscope diagram of an AChE reactor, FIG. 3E is a 521× electron microscope diagram of FIG. 3D, and FIG. 3F is a 2.00 K× electron microscope diagram of FIG. 3D;
FIG. 4A is a diagram illustrating a line fishing result of a standard mixture, and FIG. 4B is a diagram illustrating a blank control result;
FIG. 5 shows a fishing result of AChE affinity ligands in a *Mahoniae Caulis* extract, where 1 represents columbamine, 2 represents jatrorrhizine, 3 represents palmatine, and 4 represents berberine;
FIG. 6 shows LC-MS verification information of fished AChE ligands in a *Mahoniae Caulis* extract, where 1 represents columbamine, 2 represents jatrorrhizine, 3 represents palmatine, and 4 represents berberine;
FIG. 7 shows structural formulas of columbamine, jatrorrhizine, palmatine, and berberine;
FIG. 8 is a diagram illustrating a principle of an Ellman method;
FIG. 9 shows anti-AChE activity of a *Mahoniae Caulis* extract whose concentration is 128.8 µg/mL, 64.4 µg/mL, 32.2 µg/mL, 16.1 µg/mL, 8.05 µg/mL, 4.02 µg/mL, 2.01 µg/mL, 1.0 µg/mL, 0.50 µg/mL, 0.25 µg/mL, 0.125 µg/mL, and 0.063 µg/mL in sequence;
FIG. 10 shows anti-AChE effects of wild *Mahoniae Caulis,* cultivated *Mahoniae Caulis* and *Mahoniae Caulis* substitutes;
FIG. 11 is a flowchart illustrating a research on a synergistic effect of a cluster of active components; and
FIG. 12A shows synergistic effects of combined active compounds Col (A), where B1, C1, D1, E1, F1, G1, and H1 represent Col+Jat IC₃₀, Col+Pal IC₃₀, Col+Ber IC₃₀, Col+Jat IC₃₀+Pal IC₃₀, Col+Jat IC₃₀+Ber IC₃₀, Col+Pal IC₃₀+Ber IC₃₀, and Col+Jat IC₃₀+Pal IC₃₀+Ber IC₃₀, respectively; and FIG. 12B shows synergistic effects of combined active compounds Jat (B), where B2, C2, D2, E2, F2, G2, and H2 represent Jat+Col IC₃₀, Jat+Ber IC₃₀, Jat+Pal IC₃₀, Jat+Col IC₃₀+Ber IC₃₀, Jat+Col IC₃₀+Pal IC₃₀, Jat+Ber IC₃₀+Pal IC₃₀, and Jat+Col IC₃₀+Ber IC₃₀+Pal IC₃₀, respectively.

### DETAILED DESCRIPTION

To further elaborate on the technical means adopted and effects achieved in the present application, the present application is described below in conjunction with examples and drawings.

Experiments without specific techniques or conditions noted in the examples are conducted according to techniques or conditions described in the literature in the art or a product specification. The reagents or instruments used herein without manufacturers specified are conventional products commercially available from proper channels.

### Reagents and Materials

An AChE inhibitor, acetylthiocholine iodide (ATCI), 5,5'-dithiobis(2-nitrobenzoic acid) (DTNB), azobisisobutyronitrile (AIBN), ethylene glycol dimethylacrylate (EDMA), glycidyl methacrylate (GMA), 3-(isobutenoyloxy)propyltrimethoxysilane (y-MAPS), 1,4-butanediol, n-propanol, formic acid, ammonium acetate and sodium hydroxide (NaOH) were purchased from Aladdin Biochemical Technology Co., Ltd; acetylcholinesterase (AChE) was purchased from Sigma; methanol (MeOH) and acetonitrile were purchased from Merck; a capillary column (400 µm I.D.× 800 µm O.D.) was purchased from PolyMicro Technologies (Phoenix, AZ, USA); standards columbamine, jatrorrhizine hydrochloride, palmatine hydrochloride, and berberine hydrochloride were purchased from National Institutes for Food and Drug Control; cultivated *Mahoniae Caulis,* wild *Mahoniae Caulis,* and substitutes *Mahonia duclouxiana* Gagnep, *Mahonia bodinieri* Gagnep, and *Mahonia breviracema* Y.S. Wang et Hsiao were from Guangxi, and *Mahonia fordii Schneid,* was collected from Shaoguan, Guangdong and identified as genuine by Professor Deng Weixian of Shaoguan University; and water was ultrapure water.

### Instruments

A precision peristaltic pump used for an AChE immobilization experiment and an offline ligand fishing experiment was purchased from Longer Precision Pump (Baoding) Co., Ltd (Hebei, China); Jinghong DK-S22 water bath cauldron was used for preparing a matrix monolithic column through thermal initiation; a chromatograph was Agilent 1260 (Agilent Technologies, Santa Clara, CA, USA); a mass spectrometer was Agilent 6130 (Agilent Technologies, Santa Clara, CA, USA); Ultra-55 field emission scanning electron microscope (Zeiss, Germany) was used for characterizing a microstructure of the monolithic column; and Fourier transform infrared spectroscopy (FTIR) (Bruker, Germany) was used for analyzing structures of the matrix monolithic column and an enzyme reactor.

The invention is defined in the appended claims, any of the following examples that do not fall within the scope of the claims do not form part of the invention and are provided for comparative or illustrative purposes only.

### Example 1 Preparation of an AChE reactor

### (1) Pretreatment of a capillary

A quartz capillary with a length of about 2 m and an inner diameter of 400 µm was rinsed with a NaOH solution (1 moL/L) for 20 min. Both ends of the capillary filled with NaOH were sealed with rubber stoppers and a reaction was performed in a 100 °C water bath cauldron for 2 h. The capillary was taken out, rinsed with distilled water until an effluent was neutral (pH paper test), and rinsed with MeOH for 30 min. The capillary was dried with nitrogen for 2 h and then a mixed solution of γ-MAPS/MeOH (50/50, v/v) was infused into the dried capillary. The capillary was sealed with the rubber stoppers and a reaction was performed in a 60 °C water bath cauldron for 12 h. The capillary after the reaction was rinsed with methanol and distilled water for 30 min separately and then fully dried with nitrogen.

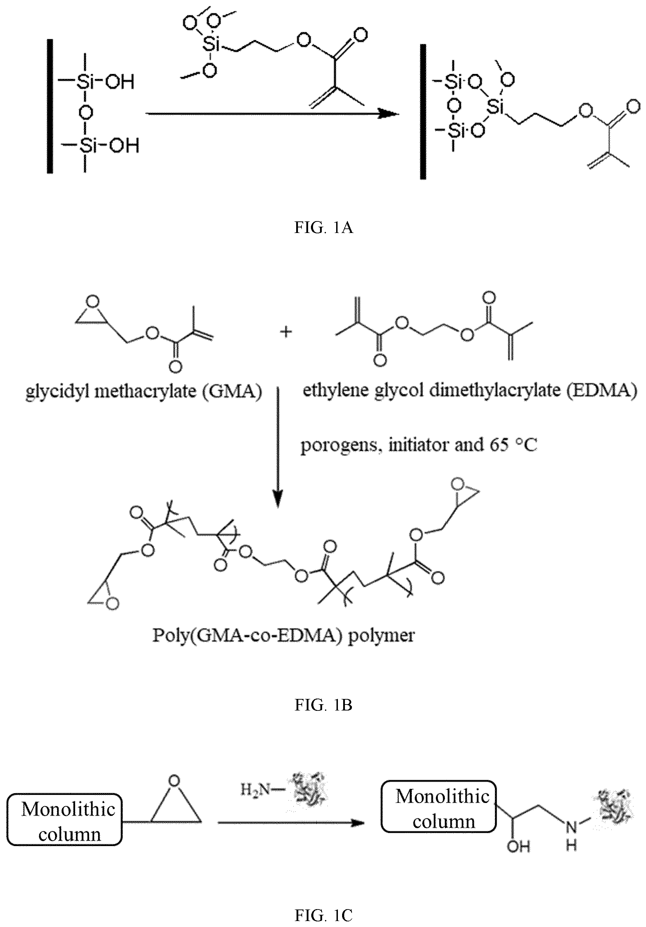

As shown in FIG. 1A, an inner wall of the quartz capillary was activated and an alkenyl group was modified on the inner wall of the capillary, which provided polymerization sites for subsequent in situ polymerization of functional monomers and a crosslinking agent.

### (2) Preparation of a poly(GMA-co-EDMA) monolithic column

As shown in FIG. 1B, referring to a method for preparing the poly(GMA-co-EDMA) monolithic column reported in the literature, the same organic polymerization system was selected, that is, GMA and EDMA were used as functional monomers, 1,4-butanediol, n-propanol, and water were used as porogens, and AIBN was used as an initiator, and components were weighed in proportion in a 1 mL glass vial and completely dissolved through ultrasonic waves so that a uniform polymerization solution was formed.

The polymerization solution was infused with nitrogen into the quartz capillary subjected to pretreatment and then the quartz capillary was sealed with rubber stoppers. The capillary was observed under a microscope to make sure that there were no bubbles in the capillary, and then a reaction was performed in a 65 °C water bath cauldron for 12 h.

After the reaction was completed, an unpolymerized reaction solution in the capillary was removed with methanol by a high-pressure pump so that the poly(GMA-co-EDMA) monolithic matrix column was prepared.

### (3) AChE immobilization

57.61 mg of chromatographic grade ammonium acetate powder was weighed in a 50 mL reagent bottle, added with distilled water, and dissolved through ultrasonic waves into a 15 mM ammonium acetate buffer whose pH was adjusted to about 8.0 with ammonia water.

1.190 mg of lyophilized AChE powder was weighed in a 5 mL glass reagent bottle, dissolved in 2 mL of ammonium acetate buffer (15 mM, pH = 7.8) through ultrasonic waves, and placed in a 4 °C refrigerator for use.

The prepared poly(GMA-co-EDMA) monolithic column was used as an enzyme immobilization carrier and the enzyme was immobilized on an inner surface of poly(GMA-co-EDMA) through a ring-opening reaction between an epoxy group on GMA and an amino group on AChE. The reaction process is shown in FIG. 1C and includes the specific steps below.

The poly(GMA-co-EDMA) monolithic column was rinsed with the ammonium acetate buffer (15 mM, pH = 7.8) for 10 min, and an AChE solution (0.5 mg/mL) was pumped into the matrix column with a constant flow syringe pump at a flowrate of 60 µL/h for enzyme immobilization for 3 h. The poly(GMA-co-EDMA) monolithic column was rinsed with distilled water for 10 min. The AChE reactor, AChE-ICER, was finally prepared and stored in a 4 °C refrigerator for use.

### Example 2 Characterization of an AChE reactor

### (1) Infrared spectrum characterization

AChE, a poly(GMA-co-EDMA) monolithic matrix material with no enzyme immobilized, and the AChE reactor, AChE-ICER, were characterized by an infrared spectrometer.

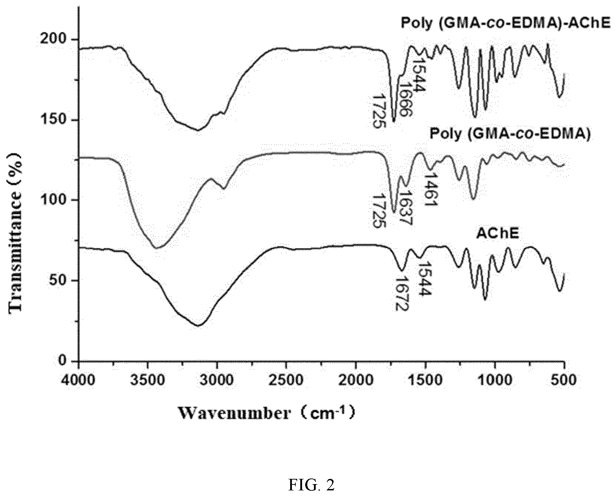

As shown in FIG. 2, 1725 cm⁻¹ is the stretching vibration absorption of the poly(GMA-co-EDAM) monolithic matrix C=O, 1544 cm⁻¹ is the bending vibration of a peptide bond N-H, 1666 cm⁻¹ is the stretching vibration absorption of C=O in the peptide bond and FT-IR results show that AChE has been successfully modified on the poly(GMA-co-EDAM) monolithic matrix carrier.

### (2) Scanning electron microscope characterization

In this example, Ultra-55 field emission scanning electron microscope was used for visually characterizing the morphology and structure of the cross-sections of the poly(GMA-co-EDMA) monolithic matrix material and the AChE reactor, AChE-ICER.

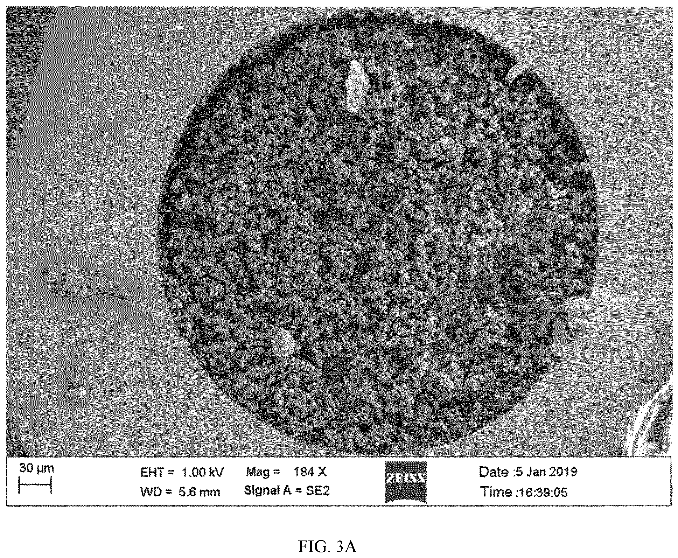

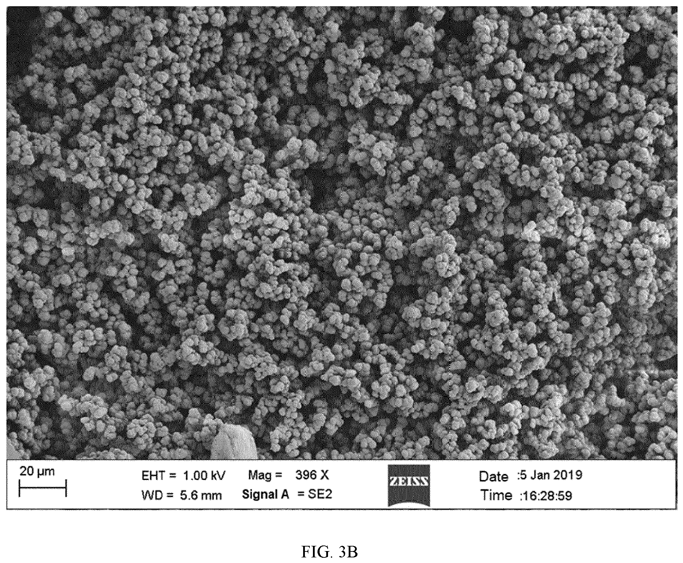

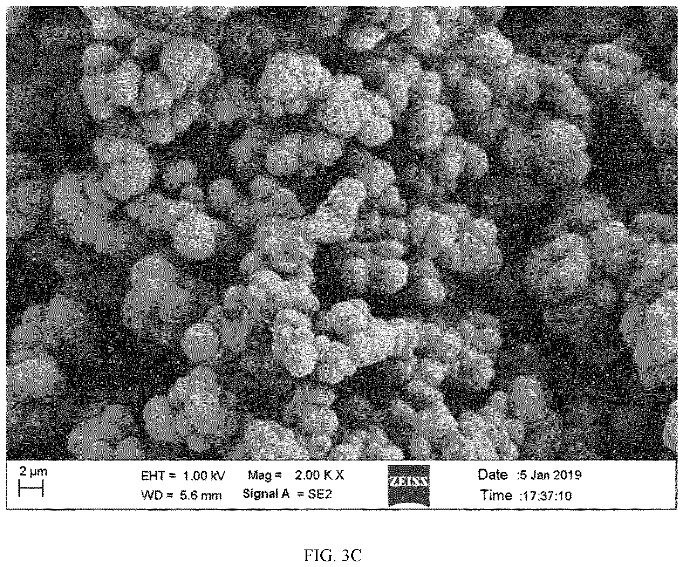

As shown in FIGS. 3A, 3B and 3C, monolithic stationary phases of the poly(GMA-co-EDAM) matrix material bind closely to an inner wall of a capillary without obvious wall-off phenomenon, and an inner polymer is uniformly distributed in the capillary in the form of microspheres and has a loose structure and excellent permeability, which is conducive to subsequent enzyme immobilization.

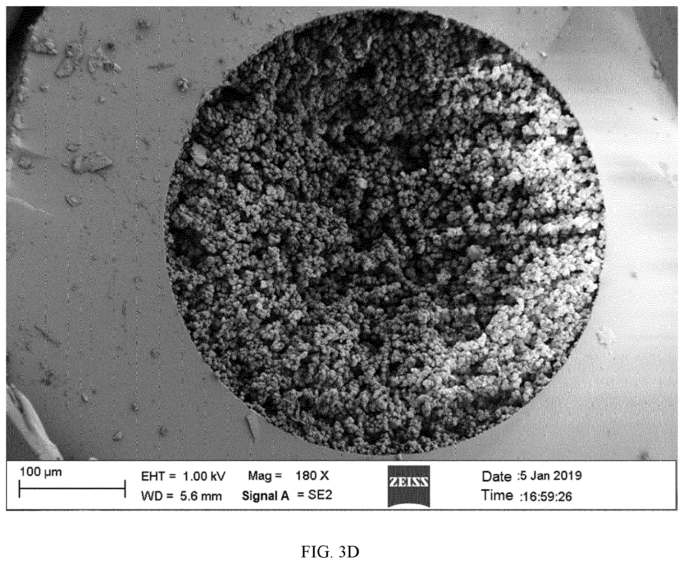

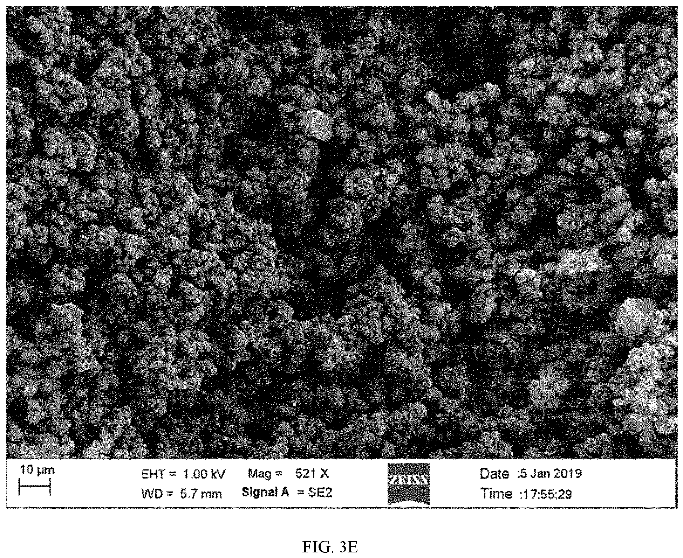

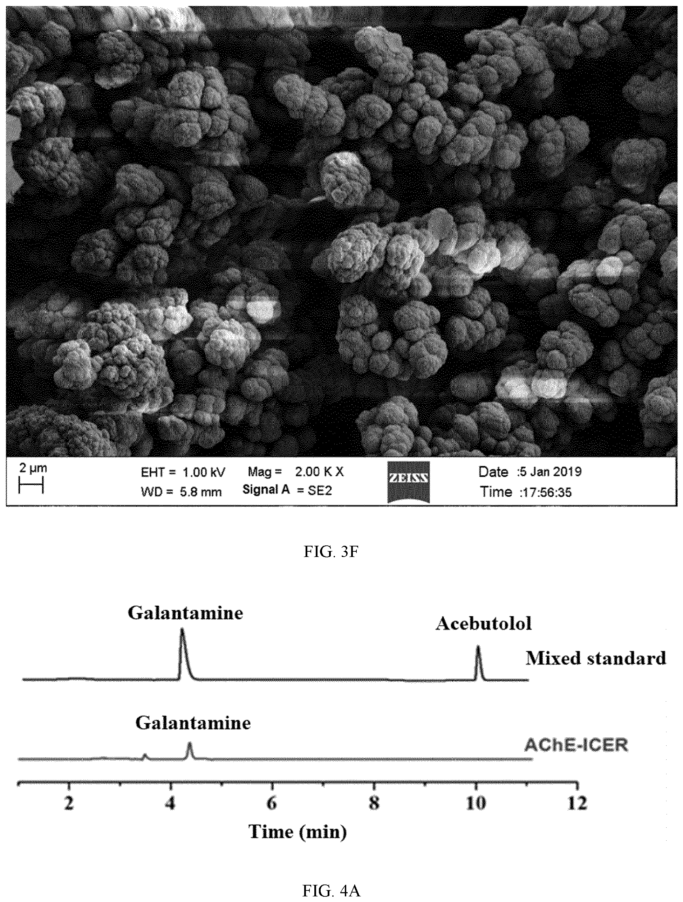

As shown in FIGS. 3D, 3E and 3F, the inner surface of the poly(GMA-co-EDAM) monolithic column becomes slightly rough after bonding AChE, but the size and uniformity of the microspheres have no significant changes, indicating that poly(GMA-co-EDAM) stationary phases are still well polymerized after bonding AChE and can be used for an online analysis.

### Example 3 Ligand fishing method

### (1) Preparation of an extract

2.0 g of crude powder of *Mahoniae Caulis* was accurately weighed and put into a conical flask with a stopper, added with 50 mL of mixed solution of hydrochloric acid-methanol (1:100, v/v), weighed with the stopper sealed, cold-soaked for 30 min, treated through ultrasonic waves (power: 300 W, frequency: 40 kHz) for 60 min, and subjected to rotary evaporation under reduced pressure so that a dry extract was obtained.

15 mg of dry extract of *Mahoniae Caulis* was accurately weighed and put into a 5 mL volumetric flask, added with an ammonium acetate buffer (10 mM, pH = 7.4), and dissolved to a scale for a concentration of 3 mg/mL. Then, a ligand fishing analysis was ready performed.

### (2) Elution method for a functional AChE-ICER monolithic column

In a first step, a mobile phase, the ammonium acetate buffer (10 mM, pH = 7.4), was provided by a peristaltic pump at a flow rate of 5 µL/min and a sample was injected at a volume of 100 µL, and the process lasted for 40 min, then fractions were collected.

In a second step, the mobile phase was changed from the ammonium acetate buffer (10 mM, pH = 7.4) to methanol and provided at a flow rate of 20 µL/min and target components were dissociated from proteins, and the process lasted for 20 min, then fractions were collected.

A blank control monolithic column, Control-ICER, was set by the same operation method and the corresponding fractions were collected.

The collected fractions were blown to dryness with N₂, dissolved in 100 µL of methanol, and analyzed through LC-MS.

LC-MS conditions: a chromatographic column Thermo Acclaim TM C18 (4.6 mm× 250 mm, 5 µm); mobile phases where acetonitrile was mobile phase A and 0.08% (v/v) formic acid and a 2 mM ammonium acetate solution were mobile phase B; a flow rate of 1.0 mL/min; an injection volume of 3 µL; a column temperature of 25 °C; and a detection wavelength of 280 nm.

Gradient elution conditions: 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B.

MS conditions: a positive ion mode, a full scan range of 100-600 m/z, a capillary voltage of 4000 V, an atomizing gas pressure of 40 psi, a dryer temperature of 350 °C, a drying gas flowrate of 10.0 L/min, and a fragmentor voltage of 100 V.

### Example 4 Ligand fishing analysis

### (1) Separation Analysis of a Standard Mixture

### A Preparation of mixed control solutions

An appropriate amount of galantamine hydrobromide and an appropriate amount of acebutolol were weighed separately as control and added with a 10% methanol solution so that a galantamine stock solution with a concentration of 1.032 mg/mL and an acebutolol stock solution with a concentration of 1.077 mg/mL were prepared.

An appropriate amount of galantamine stock solution and an appropriate amount of acebutolol stock solution were taken separately and prepared into mixed control solutions with a concentration of 0.516 mg/mL and a concentration of 0.5385 mg/mL respectively, which were filtered through 0.45 µm microporous membrane for use.

### B Chromatography conditions of mixed control solutions

The mixed control solutions of galantamine and acebutolol were analyzed by an LC-20A HPLC system. The stationary phases were Thermo Acclaim TM 120 C18 (250 mm×4.6 mm, 5 µm), and the mobile phases were acetonitrile as mobile phase A and 0.08% (v/v) formic acid and the 2 mM ammonium acetate solution as mobile phase B. The gradient elution conditions were a proportion of phase A with time: 0-4 min and 15-15%; 4-12 min, 15-45%; a flowrate of 1 mL/min, a column temperature of 25 °C, an injection volume of 5 µL, and a detection wavelength of 280 nm.

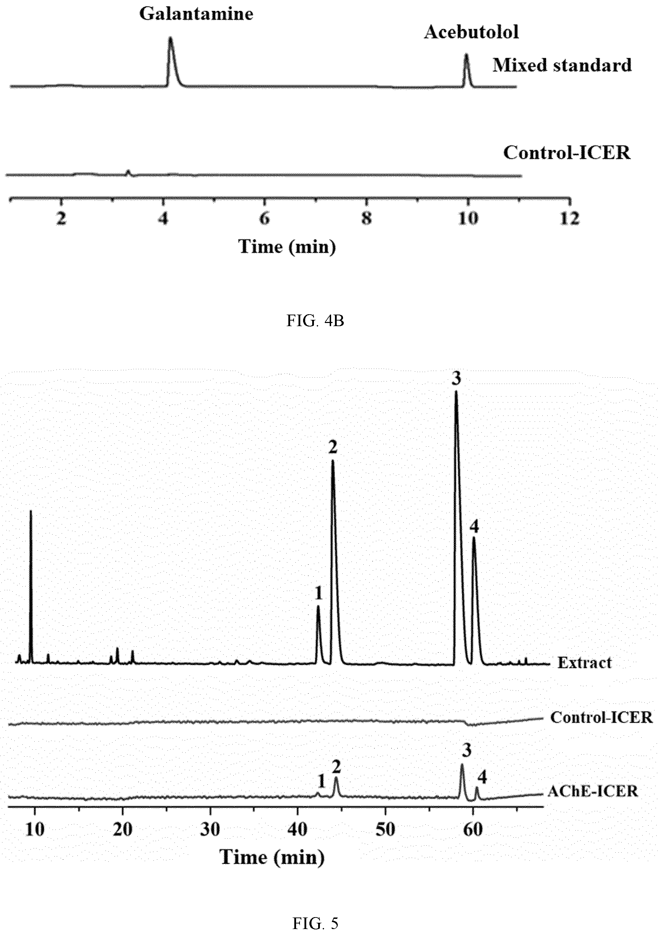

Analytical results of mixed systems composed of a negative compound acebutolol (20 µg/mL, dissolved in the ammonium acetate buffer (10 mM, pH = 7.4)) and a positive compound galantamine (20 µg/mL, dissolved in the ammonium acetate buffer (10 mM, pH = 7.4)) respectively are shown in FIGS. 4A and 4B. After standard mixtures of galantamine and acebutolol acted separately with functional AChE-ICER and enzyme-inactive Control-ICER, the compound galantamine remains only on functional AChE-ICER, while the compound acebutolol remains neither on AChE-ICER nor on enzyme-inactive Control-ICER, indicating that the prepared AChE-ICER has good selectivity for specific binding components and very weak non-specific adsorption. This proves the effectiveness of an on-line active compound fishing platform AChE-ICER, which can be further applied to on-line fishing of specific binding ligands of AChE in natural plants.

### Example 5 Screening of active components in a Mahoniae Caulis extract

The preparation of test solutions and LC-MS analysis conditions were the same as those in Example 4.

Results are shown in FIG. 5: four potential active compounds are fished from the *Mahoniae Caulis* extract through a functional AChE reactor, AChE-ICER, and the adsorption or non-specific adsorption with inactive sites of an enzyme of other compounds in the extract are excluded through a comparison with a parallel inactive AChE reactor, Control-ICER, so as to determine four compounds to be the specific binding ligands of AChE.

### Example 6 Confirmation of structures of ligands of AChE

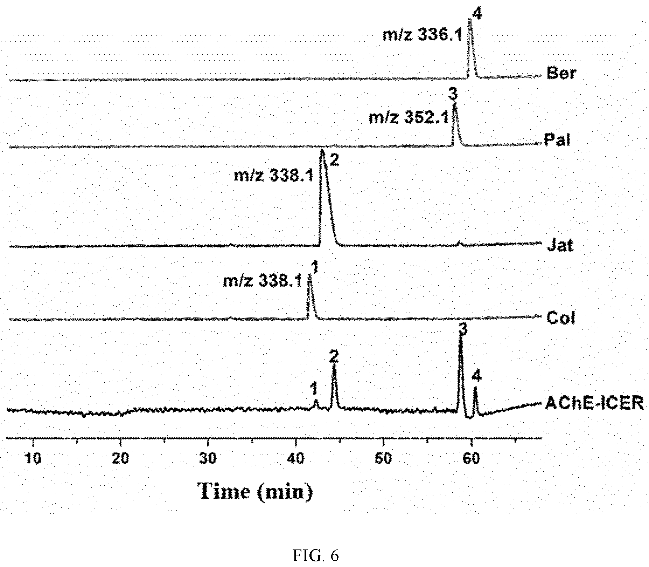

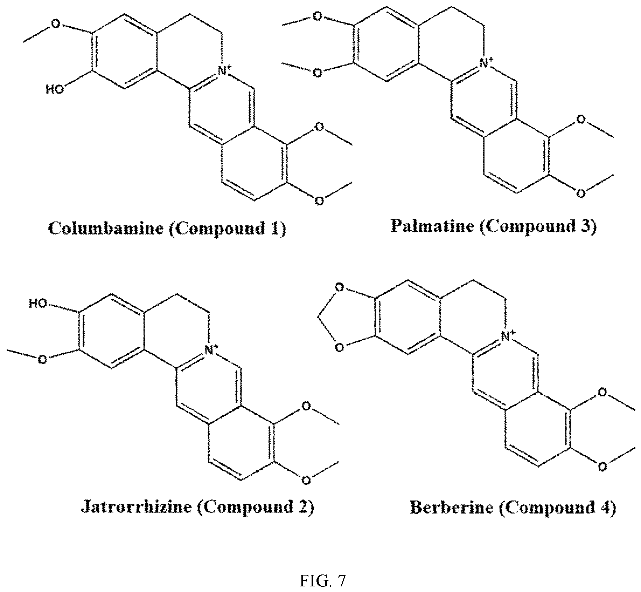

The four compounds fished in Example 5 were compared with a standard in aspects of retention time and MS data. It was confirmed that Compound 1 was columbamine (Col), Compound 2 was jatrorrhizine (Jat), Compound 3 was palmatine (Pal), and Compound 4 was berberine (Ber). The results are shown in FIGS. 6 and 7.

### Example 7 Quality control method of a Mahoniae Caulis extract established for fished ligand components

### LC-MS conditions were as described above.

Preparation of control solutions: an appropriate amount of control substance was accurately weighed, added with an appropriate amount of acetonitrile-water (3:7) mixed solution, dissolved and diluted into a mixed solution containing 20 µg per 1 mL, and then acquired.

Verification parameters of the method such as precision, recovery rate, stability, linear, range, limit of detection and limit of quantitation were investigated with reference to verification guidelines of Chinese Pharmacopoeia (Volume IV, Edition 2015). The results are shown in the table below. The linear ranges of all standard solutions are 2.94 ng/mL to 771.2 ng/mL. The correlation coefficients R² are all greater than 0.99 indicates good linearity of the method. The limit of quantitation (LOQ) ranges from 2.84 ng/mL to 5.79 ng/mL, and the limit of detection (LOD) ranges from 1.01 ng/mL to 2.07 ng/mL. The RSD of intra-day precision and the RSD of inter-day precision are less than 3.3% and 3.5%, respectively, which indicates that the method had good sensitivity and precision. The accuracy of the method was evaluated at three concentration levels: high, medium and low. Results show that the recovery rate ranges from 98.3% to 102.1% (at a high concentration), from 97.3 % to 103.1% (at a medium concentration) and from 96.3% to 98.2% (at a low concentration), indicating that the method has good accuracy. Content measurement results are shown in the following table.

**Table 1 Verification data of four ligands by an LC-MS method**

| **Analytes** | **Regression curve** | **Linea r range (ng·m** | **R²** | | **LO D (ng/ mL** | **LO Q (ng/ mL)** | **Precisio n (RSD, %, n=6)** | **Stab ility** | | **Accuracy (%)** | |
|---|---|---|---|---|---|---|---|---|---|---|---|
| | | **L⁻¹)** | | **)** | | **int ra-da y a** | **int er-da y b** | **(RS D, %, n=6)** | **hig h spi ke** | **medi um spike** | **low spik e** |
| Columba mine | Y=1190459 .6X+43915 05.4 | 5.19-3 40.0 | 0.9 99 | 1.8 6 | 5.19 | 2.8 | 3.5 | 2.7 | 98. 3 | 97.3 | 98.2 |
| Jatrorrhizi ne | Y=1351913 .3X+20733 067.0 | 5.79-7 60.0 | 0.9 95 | 2.0 7 | 5.79 | 3.3 | 2.8 | 2.6 | 10 2.1 | 103.1 | 97.9 |
| Palmatine | Y=1217798 .6X+17997 908.7 | 2.84-7 43.7 | 0.9 96 | 1.0 1 | 2.84 | 2.1 | 1.9 | 3 | 10 0.2 | 99.5 | 96.3 |
| Berberine | Y=1058506 .7X+19725 819.0 | 2.94-7 71.2 | 0.9 94 | 1.0 5 | 2.94 | 2.3 | 2.5 | 3.1 | 10 0.4 | 99.6 | 98.2 |

| | | | | | | | | | | | |
|---|---|---|---|---|---|---|---|---|---|---|---|
| ^{a}Maximum deviation within one day (based on a peak area in percentage) ^{b}Deviation within three days (based on a peak area in percentage) | | | | | | | | | | | |

**Table 2 Content of four ligands in the Mahoniae Caulis extract**

| **Sample** | **Content of alkaloids (µg/g) (n=3)** | | | |
|---|---|---|---|---|
| | **Columbamine** | **Jatrorrhizine** | **Palmatine** | **Berberine** |
| *Mahoniae Caulis* | 357.44 | 1847.78 | 3304.94 | 3576.93 |

### Example 8 Determination of anti-AChE activity of fished ligands

### (1) Preparation of solutions

ATCI (15 mM): 43.37 mg of ATCI was accurately weighed in a 10 mL volumetric flask and diluted to volume with PBS.

DTNB (3.3 mM): 1.32 mg of DTNB was accurately weighed in a 10 mL volumetric flask and diluted to volume with PBS.

AChE (3.03 U/mL): 0.55 mg of AChE was accurately weighed in a 50 mL volumetric flask and diluted to volume with PBS.

### (2) Determination of anti-AChE activity of fished ligands

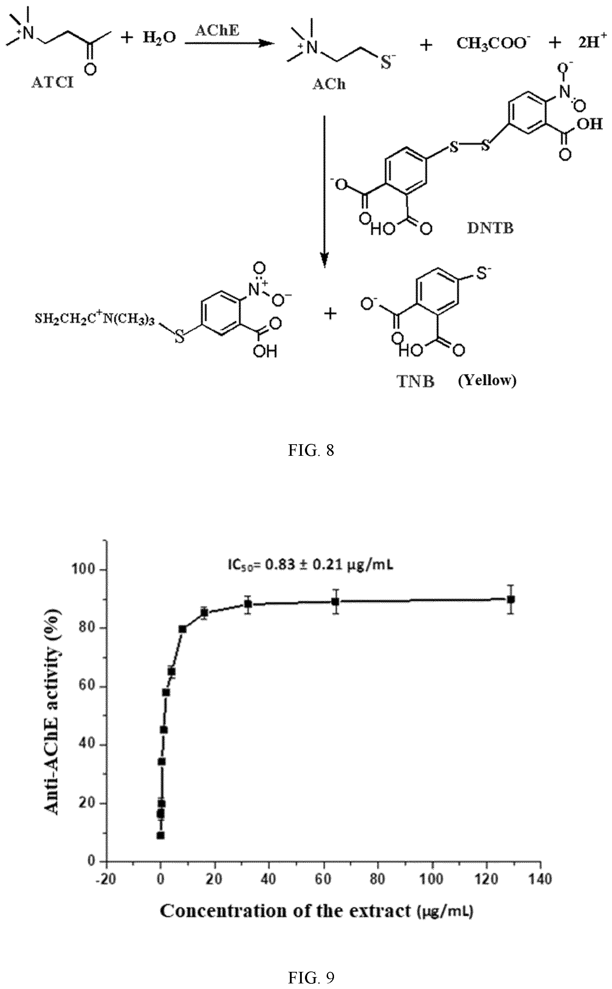

The anti-AChE activity was determined by an improved Ellman method whose principle is shown in FIG. 8. For the reaction system, see Table 3. The specific steps are described below.

140 µL of phosphate buffer (PBS, 0.1 mol/L, pH = 7.4), 20 µL of AChE (0.303 U/mL) and 20 µL of sample solution were added in sequence to a 96-well plate, mixed evenly, and added with 10 µL of DTNB (3.3 mM) and 10 µL of acetylthiocholine iodide (ATCI) (15 mM). They were mixed evenly and incubated for 10 min at 37 °C, then measured absorbance at 405 nm.

The sample was replaced with PBS in a blank enzyme group. The sample was replaced with PBS in a blank enzyme-free group. The enzyme solution was replaced with PBS in the sample control group. Galantamine was used in the positive control group.

The inhibitory rate of the sample against AChE activity is calculated by the following formula: $Inhibitory rate = \left[1-\left({A}_{sample}-{A}_{sample control}\right)/\left({A}_{blank enzyme}-{A}_{blank enzyme - free}\right)\right] \times 100 %$

The median inhibitory concentration of AChE was calculated by testing inhibitory rates at different sample concentrations. All experiments were repeated three times and means and standard deviations were calculated, where galantamine acted as the positive control.

**Table 3 Anti-AChE activity screening method**

| Addition Sequence | Group | | | |
|---|---|---|---|---|
| | Sample Group (µL) | Blank Group (µL) | Control Group (µL) | Positive Control Group (µL) |
| 1-PBS | 140 | 140+15 | 140+15+20 | 140 |
| 2-enzyme | 15 | / | / | 15 |
| 3-sample | 20 | 20 | / | 20 (Galantamine) |
| 4-ATCI | 10 | 10 | 10 | 10 |
| 5-DTNB | 10 | 10 | 10 | 10 |

FIG. 9 shows the inhibition of the *Mahoniae Caulis* extract, where IC₅₀ is 0.83±0.21 µg/mL. The activity of the positive control galantamine and the screened four compounds was evaluated by the same method. The results are as follows: the IC₅₀ of the positive control galantamine is 1.62±0.13 µg/mL, the IC₅₀ of columbamine is 0.93±0.12 µg/mL, the IC₅₀ of Jatrorrhizine is 3.50±0.15 µg/mL, the IC₅₀ of palmatine is 1.52±0.13 µg/mL, and the IC₅₀ of berberine is 2.51±0.12 µg/mL. All the four compounds exhibit different degrees of anti-AChE activity.

The *Mahoniae Caulis* extract has a stronger inhibitory effect than galantamine, jatrorrhizine, palmatine, and berberinee, which indicates that the anti-AChE activity of the *Mahoniae Caulis* extract is exerted through the synergistic effect of several active components.

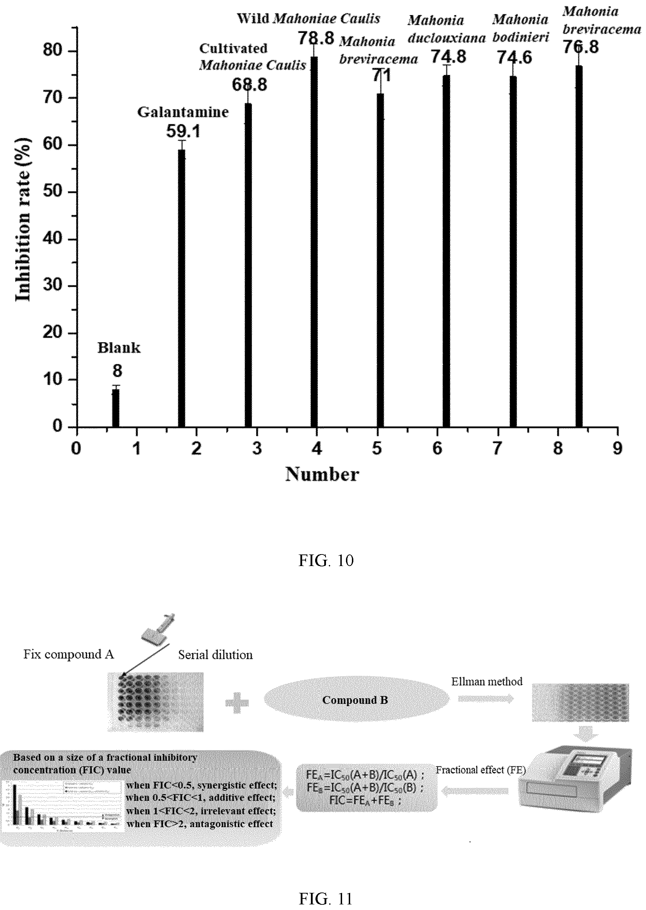

Additionally, the anti-AChE effects of wild *Mahoniae Caulis,* cultivated *Mahoniae Caulis,* and substitutes were studied at a concentration of 0.25 µg/mL, whose activity is, in a descending order, wild *Mahoniae Caulis* > cultivated *Mahoniae Caulis > Mahonia bodinieri* Gagnep *> Mahonia duclouxiana* Gagnep *> Mahonia fordii Schneid > Mahonia breviracema* Y.S. Wang et Hsiao. The results are shown in FIG. 10. This indicates that genuine *Mahoniae Caulis* and substitutes have significant differences in activity and whether the substitutes can replace *Mahoniae Caulis* for use is to be discussed.

### Example 9 Research on a synergistic effect of a cluster of active components

With reference to the method of D. Kaufmann et al. (D. Kaufmann, A.K. Dogra, A. Tahrani, F. Herrmann, M. Wink, Extracts from Traditional Chinese Medicinal Plants Inhibit Acetylcholinesterase, a Known Alzheimer's Disease Target, Molecules, 2016(21): 1161-1177.), each active monomer component was accurately weighed for 1.0 mg and dissolved with acetonitrile-water (3:7) to a concentration of 1 mg/mL. One pharmaceutical component, compound A, was selected and diluted step by step according to 1:1; the concentration of a second pharmaceutical component, compound B, was fixed to IC₃₀; based on a size of a fractional inhibitory concentration (FIC) value, the concentration of compound A was fixed, the concentration of pharmaceutical compound B was changed, and the FIC value was determined.

Fractional effects (FEs) of the two compounds are as follows: ${FE}_{A} = {IC}_{50} \left(A+B\right) / {IC}_{50} \left(A\right)$ ${FE}_{B} = {IC}_{50} \left(A+B\right) / {IC}_{50} \left(B\right)$ $FIC = {FE}_{A} + {FE}_{B}$

When the FIC is less than 0.5, the two compounds are synergistic; when FIC is 0.5 to 1, the two compounds have a superposition effect; when the FIC is 1 to 2, the two compounds are irrelevant; and when the FIC is greater than 2, the two compounds have an antagonistic effect. The route of study on their mechanism is shown in FIG. 11.

The IC₃₀ of active compounds A, B and C was calculated using SPSS 21.0 software, compound A was diluted stepwise and added to a 96-well plate, and then compound B with a fixed concentration of IC₃₀ was added to the 96-well plate, which were determined by an AChE assay method, and the FIC value was also determined.

By analogy, all possible combinations were calculated. The results are shown in Table 4.

**Table 4 Dilution concentration and fold**

| Compound A (µg/mL) | Compound B | Dilution Fold |
|---|---|---|
| 250 | IC₃₀ | 4 |
| 125 | IC₃₀ | 8 |
| 62.5 | IC₃₀ | 16 |
| 31.25 | IC₃₀ | 32 |
| 15.62 | IC₃₀ | 64 |
| 7.81 | IC₃₀ | 128 |
| 3.906 | IC₃₀ | 256 |
| 1.95 | IC₃₀ | 512 |
| 0.976 | IC₃₀ | 1024 |
| 0.488 | IC₃₀ | 2048 |
| 0.244 | IC₃₀ | 4096 |
| 0.122 | IC₃₀ | 8192 |

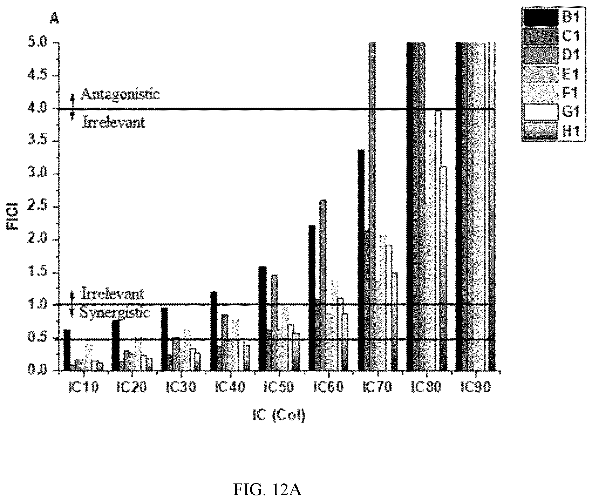

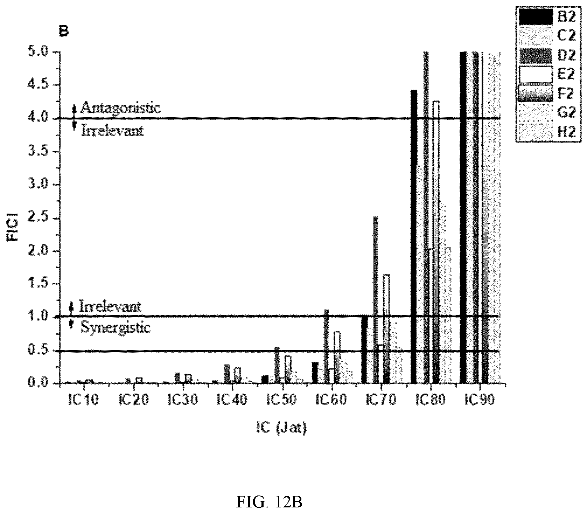

The results are shown in FIGS. 12A and 12B and Table 5. A significant synergistic effect is observed when columbamine (Col) and jatrorrhizine (Jat) are used as the first alkaloid. In some combinations using Col or Jat as the first alkaloid (below IC₆₀ or IC₇₀), the synergistic effect is exerted when the FICI is less than 0.5 and the superposition effect is exerted when the FICI is 0.5 to 1.

A combination Col IC₂₀+Jat IC₃₀+Ber IC₃₀ has the best synergistic effect (FICI = 0.49). A combination Col IC₅₀+Jat IC₃₀+Ber IC₃₀ has the best superposition effect (FICI = 0.99). As the concentration continues to increase (above IC₇₀), the compounds exhibit either an irrelevant effect or an antagonistic effect.

Additionally, when palmatine (Pal) and berberine (Ber) were used as the first alkaloid, only the irrelevant effect or the antagonistic effect was exerted. It is of great significance for the research and development of new anti-AD drugs to obtain the combination with the synergistic or superposition effect.

**Table 5 Synergistic inhibition of columbamine, jatrorrhizine, palmatine, and berberine on AChE activity**

| Sample | IC Value (Standard Deviation) | | | | | | | | |
|---|---|---|---|---|---|---|---|---|---|
| | IC₁₀ | IC₂₀ | IC₃₀ | IC₄₀ | IC₅₀ | IC₆₀ | IC₇₀ | IC₈₀ | IC₉₀ |
| Col | 0.07 (0.03) | 0.18 (0.02) | 0.34 (0.04) | 0.58 (0.10) | 0.93 (0.12) | 1.50 (0.40) | 2.54 (0.56) | 4.80 (0.78) | 12.54 (1.03) |
| **Col** + **Jat** IC₃₀ | 0.04 (0.02) | 0.12 (0.03) | 0.24 (0.05) | 0.43 (0.06) | 0.74 (0.07) | 1.26 (0.12) | 2.27 (0.21) | 4.63 (1.23) | 13.62 (2.10) |
| FICI | 0.62 | 0.77 | 0.96 | 1.21 | 1.59 | 2.21 | 3.36 | 6.01 | 15.93 |
| Col +Pal IC₃₀ | 0.01 (0.01) | 0.02 (0.01) | 0.05 (0.02) | 0.11 (0.06) | 0.23 (0.05) | 0.48 (0.12) | 1.07 (0.36) | 2.80 (0.87) | 11.84 (2.30) |
| FICI | **0.08** | **0.14** | **0.24** | **0.38** | **0.62** | 1.09 | 2.12 | 5.04 | 19.80 |
| Col +Ber IC₃₀ | 0.01 (0.01) | 0.03 (0.01) | 0.08 (0.03) | 0.17 (0.05) | 0.32 (0.06) | 0.63 (0.13) | 1.30 (0.14) | 3.15 (1.02) | 11.94 (3.2) |
| FICI | **0.16** | **0.30** | **0.51** | **0.86** | 1.46 | 2.59 | 5.00 | 11.55 | 42.26 |
| Col +Jat IC₃₀+Pal IC₃₀ | 0.01 (0.01) | 0.04 (0.01) | 0.10 (0.02) | 0.21 (0.04) | 0.41 (0.10) | 0.81 (0.20) | 1.69 (1.10) | 4.14 (1.60) | 15.90 (2.50) |
| FICI | **0.16** | **0.25** | **0.34** | **0.45** | **0.61** | **0.87** | 1.35 | 2.54 | 7.73 |
| Col +Jat IC₃₀+Ber IC₃₀ | 0.03 (0.01) | 0.08 (0.05) | 0.16 (0.07) | 0.30 (0.1) | 0.53 (0.2) | 0.92 (0.3) | 1.68 (0.8) | 3.52 (1.02) | 10.73 (3.0) |
| FICI | **0.40** | **0.49** | **0.62** | **0.77** | **0.99** | 1.37 | 2.06 | 3.67 | 9.80 |
| Col +Pal | 0.01 | 0.04 | 0.08 | 0.17 | 0.33 | 0.63 | 1.28 | 3.04 | 11.18 |
| IC₃₀+Ber IC₃₀ | (0.01) | (0.01) | (0.02) | (0.06) | (0.02) | (0.12) | (0.56) | (1.21) | (2.89) |
| FICI | **0.16** | **0.23** | **0.34** | **0.48** | **0.71** | 1.11 | 1.91 | 3.97 | 13.18 |
| Col +Jat IC₃₀+Pal IC₃₀+Ber IC₃₀ | 0.01 (0.01) | 0.03 (0.01) | 0.08 (0.03) | 0.17 (0.09) | 0.35 (0.12) | 0.71 (0.21) | 1.57 (0.36) | 4.11 (1.20) | 17.45 (5.01) |
| FICI | **0.11** | **0.18** | **0.26** | **0.38** | **0.57** | **0.87** | 1.48 | 3.10 | 10.93 |
| Jat | 0.11 (0.05) | 0.39 (0.60) | 0.92 (0.51) | 1.86 (0.89) | 3.50 (0.15) | 6.65 (1.10) | 13.38 (3.20) | 31.36 (4.20) | 63.20 (5.60) |
| Jat + Col IC₃₀ | 0.001 (0.001) | 0.002 (0.001) | 0.01 (0.01) | 0.03 (0.01) | 0.08 (0.02) | 0.25 (0.05) | 0.87 (0.12) | 3.94 (0.23) | 38.01 (4.20) |
| FICI | **0.01** | **0.01** | **0.02** | **0.04** | **0.11** | **0.32** | 1.02 | 4.42 | 42.00 |
| Jat + Ber IC₃₀ | 0.001 (0.001) | 0.003 (0.001) | 0.01 (0.01) | 0.03 (0.01) | 0.07 (0.02) | 0.22 (0.10) | 0.71 (0.20) | 2.93 (1.10) | 26.08 (3.20) |
| FICI | **0.01** | **0.01** | **0.02** | **0.04** | **0.10** | **0.27** | **0.82** | 3.28 | 28.82 |
| Jat + Pal IC₃₀ | 0.01 (0.01) | 0.03 (0.01) | 0.08 (0.03) | 0.18 (0.05) | 0.41 (0.10) | 0.91 (0.12) | 2.17 (0.21) | 6.29 (1.12) | 16.80 (2.14) |
| FICI | **0.05** | **0.09** | **0.17** | **0.30** | **0.56** | 1.12 | 2.52 | 7.05 | 18.57 |
| Jat + Col IC₃₀+Ber IC₃₀ | 0.001 (0.001) | 0.002 (0.001) | 0.01 (0.01) | 0.03 (0.01) | 0.07 (0.03) | 0.18 (0.06) | 0.50 (0.12) | 1.81 (0.31) | 12.43 (1.20) |
| FICI | **0.01** | **0.01** | **0.02** | **0.04** | **0.09** | **0.22** | **0.59** | 2.03 | 13.74 |
| Jat + Col IC₃₀+Pal IC₃₀ | 0.01 (0.01) | 0.02 (0.01) | 0.06 (0.02) | 0.14 (0.06) | 0.29 (0.12) | 0.61 (0.21) | 1.39 (0.32) | 3.79 (0.45) | 16.96 (1.12) |
| FICI | **0.05** | **0.08** | **0.13** | **0.22** | **0.40** | **0.76** | 1.62 | 4.25 | 18.75 |
| Jat + Ber IC₃₀+Pal IC₃₀ | 0.001 (0.001) | 0.01 (0.01) | 0.02 (0.01) | 0.06 (0.03) | 0.13 (0.05) | 0.31 (0.09) | 0.78 (0.07) | 2.46 (0.12) | 13.75 (2.10) |
| FICI | **0.01** | **0.03** | **0.05** | **0.09** | **0.18** | **0.38** | **0.91** | 2.75 | 15.20 |
| Jat + Col IC₃₀+Ber IC₃₀+Pal | 0.001 (0.001) | 0.002 (0.001) | 0.01 (0.01) | 0.02 (0.01) | 0.05 (0.02) | 0.15 (0.06) | 0.46 (0.09) | 1.83 (0.16) | 14.55 (0.89) |
| IC₃₀ | | | | | | | | | |
| FICI | 0.01 | 0.01 | 0.01 | 0.03 | 0.07 | 0.19 | 0.54 | 2.05 | 16.07 |
| Ber | 0.07 (0.02) | 0.26 (0.12) | 0.63 (0.21) | 1.29 (0.26) | 2.51 (0.12) | 4.86 (0.34) | 9.99 (0.89) | 24.08 (1.12) | 50.20 (2.40) |
| Ber + Col IC₃₀ | 0.07 (0.01) | 0.25 (0.10) | 0.58 (0.06) | 1.15 (0.10) | 2.13 (0.13) | 3.97 (1.12) | 7.85 (2.10) | 17.89 (3.10) | 62.05 (4.56) |
| FICI | 1.27 | 1.72 | 2.63 | 4.24 | 7.11 | 12.47 | 23.81 | 53.20 | 183.19 |
| Ber + Jat IC₃₀ | 0.23 (0.05) | 0.61 (0.12) | 1.16 (0.21) | 1.95 (0.19) | 3.15 (0.25) | 5.08 (1.10) | 8.55 (1.21) | 16.14 (2.30) | 42.00 (3.12) |
| FICI | 3.61 | 3.02 | 3.11 | 3.64 | 4.69 | 6.58 | 10.17 | 18.26 | 46.59 |
| Ber + Pal IC30 | 0.20 (0.05) | 0.50 (0.09) | 0.93 (0.11) | 1.55 (0.16) | 2.18 (0.19) | 3.97 (0.79) | 6.62 (0.86) | 12.35 (2.10) | 31.58 (3.20) |
| FICI | 3.50 | 3.63 | 4.70 | 6.56 | 8.41 | 14.54 | 23.55 | 43.24 | 109.92 |
| Ber + Col IC₃₀+Jat IC₃₀ | 0.15 (0.05) | 0.39 (0.11) | 0.73 (0.09) | 1.24 (0.25) | 2.00 (0.19) | 3.24 (0.18) | 5.47 (0.32) | 10.39 (0.79) | 27.23 (1.25) |
| FICI | 2.25 | 1.78 | 1.75 | 1.94 | 2.40 | 3.26 | 4.93 | 8.74 | 22.33 |
| Ber + Col IC₃₀+Pal IC₃₀ | 0.10 (0.03) | 0.29 (0.05) | 0.59 (0.10) | 1.05 (0.13) | 1.78 (0.19) | 3.02 (0.89) | 5.39 (1.21) | 10.82 (1.56) | 31.06 (2.10) |
| FICI | 1.64 | 1.59 | 1.88 | 2.48 | 3.54 | 5.42 | 9.09 | 17.62 | 49.93 |
| Ber + Jat IC₃₀+Pal IC₃₀ | 0.21 (0.10) | 0.52 (0.21) | 0.96 (0.32) | 1.56 (0.35) | 2.46 (0.49) | 3.86 (1.10) | 6.33 (1.25) | 11.54 (2.69) | 28.53 (3.50) |
| FICI | 3.25 | 2.44 | 2.32 | 2.51 | 3.03 | 4.02 | 5.91 | 10.10 | 24.34 |
| Ber + Col IC₃₀+Jat IC₃₀+Pal IC₃₀ | 0.13 (0.02) | 0.36 (0.10) | 0.73 (0.14) | 1.28 (0.25) | 2.16 (0.79) | 3.64 (0.98) | 6.44 (1.12) | 12.89 (1.56) | 36.66 (3.21) |
| FICI | 1.92 | 1.62 | 1.63 | 1.82 | 2.27 | 3.12 | 4.82 | 8.91 | 24.53 |
| Pal | 0.02 (0.01) | 0.10 (0.20) | 0.29 (0.14) | 0.69 (0.12) | 1.52 (0.13) | 3.35 (0.52) | 7.96 (0.89) | 22.84 (1.25) | 48.30 (3.21) |
| Pal + Col IC₃₀ | 0.11 (0.02) | 0.36 (0.12) | 0.82 (0.23) | 1.58 (0.56) | 2.89 (1.21) | 5.29 (2.31) | 10.28 (2.89) | 22.87 (3.59) | 76.75 (8.77) |
| FICI | 5.51 | 4.67 | 5.21 | 6.92 | 10.37 | 17.08 | 31.43 | 68.06 | 226.66 |
| Pal + Jat IC₃₀ | 0.59 (0.12) | 1.36 (0.21) | 2.36 (0.29) | 3.70 (0.89) | 5.61 (0.96) | 8.50 (1.10) | 13.35 (2.10) | 23.19 (3.25) | 53.19 (4.12) |
| FICI | 28.79 | 14.91 | 10.72 | 9.43 | 9.81 | 11.79 | 16.22 | 26.27 | 59.04 |
| Pal + Ber IC₃₀ | 0.10 (0.01) | 0.38 (0.12) | 0.89 (0.15) | 1.82 (0.23) | 3.48 (1.21) | 6.67 (2.06) | 13.55 (4.10) | 32.17 (5.20) | 65.80 (7.98) |
| FICI | 5.02 | 4.32 | 4.52 | 5.54 | 7.84 | 12.61 | 23.27 | 52.63 | 106.14 |
| Pal + Col IC₃₀+Jat IC₃₀ | 0.19 (0.09) | 0.55 (0.10) | 1.10 (0.16) | 1.94 (0.25) | 3.28 (0.32) | 5.55 (1.10) | 9.82 (2.31) | 19.72 (5.02) | 56.27 (8.59) |
| FICI | 9.30 | 5.85 | 4.68 | 4.39 | 4.79 | 6.09 | 9.09 | 16.64 | 46.18 |
| Pal + Col IC₃₀+Ber IC₃₀ | 0.22 (0.10) | 0.61 (0.21) | 1.20 (0.89) | 2.09 (0.78) | 3.46 (1.02) | 5.76 (2.10) | 10.00 (2.12) | 19.65 (2.56) | 54.24 (4.56) |
| FICI | 10.75 | 6.69 | 5.40 | 5.21 | 5.89 | 7.71 | 11.67 | 21.33 | 57.63 |
| Pal + Jat IC₃₀+Ber IC₃₀ | 0.26 (0.01) | 0.76 (0.12) | 1.54 (0.21) | 2.75 (1.02) | 4.69 (1.23) | 7.99 (2.10) | 14.29 (3.25) | 29.03 (4.52) | 84.35 (7.90) |
| FICI | 12.60 | 7.99 | 6.32 | 5.80 | 6.14 | 7.58 | 11.08 | 20.12 | 56.52 |
| Pal + Col IC₃₀+Ber IC₃₀+Jat IC₃₀ | 0.13 (0.02) | 0.43 (0.12) | 0.94 (0.21) | 1.78 (0.15) | 3.21 (1.21) | 5.78 (2.10) | 10.99 (3.12) | 24.06 (4.12) | 78.22 (6.89) |
| FICI | 6.31 | 4.46 | 3.74 | 3.54 | 3.82 | 4.80 | 7.23 | 13.85 | 43.23 |

To sum up, in the present application, with the traditional Chinese medicine *Mahoniae Caulis* as a study object, on the basis of an affinity chromatography principle in combination with an immobilized enzyme technology, an AChE reactor is prepared, an active compound fishing platform is constructed, four anti-AChE active components are successfully screened and verified, and an efficient screening method is provided for discovery of anti-AChE active substances in a *Mahoniae Caulis* extract. The active components are verified in vitro and their IC₅₀ is compared with the IC₅₀ of the extract, which indicates that the *Mahoniae Caulis* extract exerts a stronger anti-AChE effect than each monomer. Additionally, through the research on the synergistic effect, it is found that the four active monomer components exhibit the synergistic or superposition effect within a certain range when exerting the anti-AChE effect. The combinations having the best synergistic and superposition effects are Col IC₂₀+Jat IC₃₀+Ber IC₃₀ and Col IC₅₀+Jat IC₃₀+Ber IC₃₀, respectively. The obtained combinations having the synergistic or superposition effect can be developed into potential anti-AChE inhibitors.

## Claims

1. A pharmaceutical composition, comprising a composition having anti-AChE activity, wherein:
(a) the composition having anti-AChE activity comprises columbamine, jatrorrhizine, palmatine, and berberine;
(b) the pharmaceutical composition further comprises any one or a combination of at least two of a pharmaceutically acceptable carrier, excipient, or diluent; and
(i) the pharmaceutical composition is in a liquid form, and the columbamine, jatrorrhizine, and palmatine in the pharmaceutical composition have a concentration of 0.18 µg/mL, a concentration of 0.92 µg/mL, and a concentration of 0.63 µg/mL, respectively, components of the composition have a best synergistic effect, FICI = 0.49; or
(ii) the pharmaceutical composition is in a liquid form, and the columbamine, jatrorrhizine, and palmatine in the pharmaceutical composition have a concentration of 0.93 µg/mL, a concentration of 0.92 µg/mL, and a concentration of 0.63 µg/mL, respectively, components of the composition have a best superposition effect, FICI=0.99.

2. The pharmaceutical composition according to claim 1, wherein in the composition having anti-AChE activity,
(a) a mass ratio of the columbamine, jatrorrhizine, palmatine, and berberine is 1:5.16:9.24:10; or
(b) a mass ratio of the columbamine, jatrorrhizine, palmatine, and berberine is 0.9-1.1:4.644-5.676:8.316-10.164:9-11, and the activity of the composition is 90% to 110% of activity of a composition containing columbamine, jatrorrhizine, palmatine, and berberine whose mass ratio is 1:5.16:9.24:10.

3. A screening method for the composition having anti-AChE activity in the pharmaceutical composition according to claim 1 or 2, comprising the following steps:
(1) preparing a reactor: immobilizing AChE on a poly(GMA-co-EDMA) monolithic column;
(2) performing a ligand fishing analysis: passing a Mahoniae Caulis sample through the column in the reactor in step (1) and eluting the Mahoniae Caulis sample, and collecting fractions for LC-MS to obtain a specific binding ligand; and
(3) identifying a ligand structure: comparing the specific binding ligand obtained in step (2) with a standard in aspects of retention time and MS data to identify the ligand structure and obtain the composition.

4. The method according to claim 3, wherein a method for preparing the reactor in step (1) comprises modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column.

5. The method according to claim 3 or 4, wherein the liquid chromatography-mass spectrometry in step (2) uses acetonitrile as a mobile phase A and uses a mixture of formic acid and ammonium acetate as a mobile phase B.

6. The method according to claim 5, wherein the mobile phase A and the mobile phase B each have a flow rate of 0.5-1.5 mL/min;
preferably, the LC-MS in step (2) has an injection volume of 2-4 µL, a column temperature of 24-26 °C, and a detection wavelength of 280 nm;
preferably, gradient elution conditions of the LC-MS in step (2) comprise 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B.

7. The method according to any one of claims 3 to 6, wherein the standard in step (3) comprises a columbamine standard, a jatrorrhizine hydrochloride standard, a palmatine hydrochloride standard, and a berberine hydrochloride standard.

8. The method according to any one of claims 3 to 7, wherein, after step (3), further comprising a step of determining anti-AChE activity of the ligand;
preferably, the step of determining the anti-AChE activity of the ligand comprises mixing AChE with the ligand, adding dithiobis-nitrobenzoic acid and ATCI, measuring absorbance after incubation, and calculating an AChE inhibition rate.

9. The method according to claim 8, wherein the AChE has a final concentration of 0.0301-0.0305 U/mL;
preferably, the dithiobis-nitrobenzoic acid has a final concentration of 0.32-0.34 mM; preferably, the ATCI has a final concentration of 1.4-1.6 mM;
preferably, the incubation is performed at a temperature of 35-38 °C; and
preferably, the incubation is performed for 5-15 min.

10. The method according to any one of claims 3 to 9, comprising the following steps:
(1) preparing the reactor: modifying an alkenyl group on an inner wall of a capillary, preparing the poly(GMA-co-EDMA) monolithic column with glycidyl methacrylate and ethylene glycol dimethylacrylate as functional monomers, and immobilizing AChE on the poly(GMA-co-EDMA) monolithic column to obtain the reactor;
(2) performing the ligand fishing analysis: passing the Mahoniae Caulis sample through the column in the reactor in step (1) and eluting the Mahoniae Caulis sample, and collecting the fractions for the LC-MS to obtain the specific binding ligand, wherein the LC-MS uses acetonitrile as a mobile phase A and uses a mixture of formic acid and ammonium acetate as a mobile phase B, wherein the mobile phase A and the mobile phase B each have a flowrate of 0.5-1.5 mL/min; the LC-MS has an injection volume of 2-4 µL, a column temperature of 24-26 °C, and a detection wavelength of 280 nm; and gradient elution conditions of the LC-MS comprise 0-17 min and 5%B-18%B; 17-35 min and 18%B-21%B; 35-45 min and 21%B; 45-55 min and 21%B-25%B; 55-65 min and 25%B-50%B; or 65-90 min and 50%B-70%B;
(3) identifying the ligand structure: comparing the specific binding ligand obtained in step (2) with a columbamine standard, a jatrorrhizine hydrochloride standard, a palmatine hydrochloride standard, and a berberine hydrochloride standard in aspects of retention time and mass spectrometry data to identify the ligand structure; and
(4) determining activity of the ligand: mixing AChE with the ligand, adding dithiobis-nitrobenzoic acid and acetylthiocholine iodide, measuring absorbance after incubation for 5-15 min at 35-38 °C, and calculating an AChE inhibition rate.

11. The pharmaceutical composition according to claim 1 or 2 for use in the treatment of Alzheimer's disease (AD).

## Patentansprüche

1. Pharmazeutische Zusammensetzung, umfassend eine Zusammensetzung, die eine Anti-AChE-Aktivität aufweist, wobei:
(a) wobei die Zusammensetzung, die eine Anti-AChE-Aktivität aufweist, Columbamin, Jatrorrhizin, Palmatin und Berberin umfasst;
(b) die pharmazeutische Zusammensetzung ferner einen pharmazeutisch akzeptablen Träger, Hilfsstoff oder ein pharmazeutisch akzeptables Verdünnungsmittel oder eine Kombination von mindestens zwei davon umfasst und
(i) die pharmazeutische Zusammensetzung in einer flüssigen Form vorliegt und das Columbamin, Jatrorrhizin und Palmatin in der pharmazeutischen Zusammensetzung jeweils eine Konzentration von 0,18 µg/mL, eine Konzentration von 0,92 µg/mL und eine Konzentration von 0,63 µg/mL aufweisen, bei denen die Bestandteile der Zusammensetzung die beste synergetische Wirkung, FICI = 0,49, aufweisen, oder
(ii) die pharmazeutische Zusammensetzung eine flüssige Form aufweist und das Columbamin, Jatrorrhizin und Palmatin in der pharmazeutischen Zusammensetzung jeweils eine Konzentration von 0,93 µg/mL, eine Konzentration von 0,92 µg/mL und eine Konzentration von 0,63 µg/mL aufweisen, bei denen die Bestandteile der Zusammensetzung die beste Überlagerungswirkung, FICI = 0,99, aufweisen.

2. Pharmazeutische Zusammensetzung nach Anspruch 1, wobei in der Zusammensetzung, die eine Anti-AChE-Aktivität aufweist,
(a) ein Massenverhältnis von Columbamin, Jatrorrhizin, Palmatin und Berberin 1:5,16:9,24:10 beträgt oder
(b) ein Massenverhältnis von Columbamin, Jatrorrhizin, Palmatin und Berberin 0,9-1,1:4,644-5,676:8,316-10,164:9-11 beträgt und die Aktivität der Zusammensetzung 90 % bis 110 % der Aktivität einer Zusammensetzung beträgt, die Columbamin, Jatrorrhizin, Palmatin und Berberin enthält, deren Massenverhältnis 1:5,16:9,24:10 beträgt.

3. Screeningverfahren für die Zusammensetzung, die eine Anti-AChE-Aktivität in der pharmazeutischen Zusammensetzung aufweist, nach Anspruch 1 oder 2, umfassend die folgenden Schritte:
(1) Herstellen eines Reaktors: Immobilisieren von AChE auf einer monolithischen Poly(GMA-*co*-EDMA)-Säule;
(2) Durchführen einer Ligandenbindungsanalyse: Leiten einer Mahoniae-Caulis-Probe durch die Säule in dem Reaktor in Schritt (1) und Eluieren der Mahoniae-Caulis-Probe und Sammeln von Fraktionen für die LC-MS, um einen spezifischen bindenden Liganden zu erhalten, und
(3) Identifizieren einer Ligandenstruktur: Vergleichen des in Schritt (2) erhaltenen spezifischen bindenden Liganden mit einem Standard hinsichtlich Retentionszeit und MS-Daten, um die Ligandenstruktur zu identifizieren und die Zusammensetzung zu erhalten.

4. Verfahren nach Anspruch 3, wobei ein Verfahren zum Herstellen des Reaktors in Schritt (1) das Ändern einer Alkenylgruppe an einer Innenwand einer Kapillare, das Herstellen der monolithischen Poly(GMA-*co*-EDMA)-Säule mit Glycidylmethacrylat und Ethylenglykoldimethacrylat als funktionelle Monomere und das Immobilisieren von AChE auf der monolithischen Poly(GMA-*co*-EDMA)-Säule umfasst.

5. Verfahren nach Anspruch 3 oder 4, wobei die Flüssigchromatographie-Massenspektrometrie in Schritt (2) Acetonitril als mobile Phase A verwendet und ein Gemisch aus Ameisensäure und Ammoniumacetat als mobile Phase B verwendet.

6. Verfahren nach Anspruch 5, wobei die mobile Phase A und die mobile Phase B jeweils eine Flussrate von 0,5-1,5 mL/min aufweisen,
wobei die LC-MS in Schritt (2) vorzugsweise ein Injektionsvolumen von 2-4 µL, eine Säulentemperatur von 24-26 °C und eine Detektionswellenlänge von 280 nm aufweist,
wobei Gradientenelutionsbedingungen der LC-MS in Schritt (2) vorzugsweise 0-17 min und 5 % B-18 % B, 17-35 min und 18 % B-21 % B, 35-45 min und 21 % B, 45-55 min und 21 % B-25 % B, 55-65 min und 25 % B-50 % B oder 65-90 min und 50 % B-70 % B umfassen.

7. Verfahren nach einem der Ansprüche 3 bis 6, wobei der Standard in Schritt (3) einen Columbamin-Standard, einen Jatrorrhizinhydrochlorid-Standard, einen Palmatinhydrochlorid-Standard und einen Berberinhydrochlorid-Standard umfasst.

8. Verfahren nach einem der Ansprüche 3 bis 7, das nach Schritt (3) ferner einen Schritt zum Bestimmen einer Anti-AChE-Aktivität des Liganden umfasst,
wobei der Schritt zum Bestimmen der Anti-AChE-Aktivität des Liganden vorzugsweise das Mischen von AChE mit dem Liganden, das Zusetzen von Dithiobisnitrobenzoesäure und ATCI, das Messen der Absorption nach der Inkubation und das Berechnen einer AChE-Hemmungsrate umfasst.

9. Verfahren nach Anspruch 8, wobei die AChE eine Endkonzentration von 0,0301-0,0305 U/mL aufweist,
wobei die Dithiobisnitrobenzoesäure vorzugsweise eine Endkonzentration von 0,32-0,34 mM aufweist,
wobei das ATCI vorzugsweise eine Endkonzentration von 1,4-1,6 mM aufweist, wobei die Inkubation vorzugsweise bei einer Temperatur von 35-38 °C durchgeführt wird und
die Inkubation vorzugsweise für 5-15 min durchgeführt wird.

10. Verfahren nach einem der Ansprüche 3 bis 9, umfassend die folgenden Schritte:
(1) Herstellen des Reaktors: Ändern einer Alkenylgruppe an einer Innenwand einer Kapillare, Herstellen der monolithischen Poly(GMA-*co*-EDMA)-Säule mit Glycidylmethacrylat und Ethylenglykoldimethacrylat als funktionelle Monomere und Immobilisieren von AChE auf der monolithischen Poly(GMA-*co*-EDMA)-Säule, um den Reaktor zu erhalten;
(2) Durchführen einer Ligandenbindungsanalyse: Leiten der Mahoniae-Caulis-Probe durch die Säule in dem Reaktor in Schritt (1) und Eluieren der Mahoniae-Caulis-Probe und Sammeln der Fraktionen für die LC-MS, um den spezifischen bindenden Liganden zu erhalten, wobei die LC-MS Acetonitril als mobile Phase A verwendet und ein Gemisch aus Ameisensäure und Ammoniumacetat als mobile Phase B verwendet, wobei die mobile Phase A und die mobile Phase B jeweils eine Flussrate von 0,5-1,5 mL/min aufweisen, wobei die LC-MS ein Injektionsvolumen von 2-4 µL, eine Säulentemperatur von 24-26 °C und eine Detektionswellenlänge von 280 nm aufweist und Gradientenelutionsbedingungen der LC-MS 0-17 min und 5 % B-18 % B, 17-35 min und 18 % B-21 % B, 35-45 min und 21 % B, 45-55 min und 21 % B-25 % B, 55-65 min und 25 % B-50 % B oder 65-90 min und 50 % B-70 % B umfassen;
(3) Identifizieren der Ligandenstruktur: Vergleichen des in Schritt (2) erhaltenen spezifischen bindenden Liganden mit einem Columbamin-Standard, einem Jatrorrhizinhydrochlorid-Standard, einem Palmatinhydrochlorid-Standard und einem Berberinhydrochlorid-Standard hinsichtlich Retentionszeit und Massenspektrometriedaten, um die Ligandenstruktur zu identifizieren, und
(4) Bestimmen einer Aktivität des Liganden: Mischen von AChE mit dem Liganden, Zusetzen von Dithiobisnitrobenzoesäure und Acetylthiocholin-Iodid, Messen der Absorption nach der Inkubation für 5-15 min bei 35-38 °C und Berechnen einer AChE-Hemmungsrate.

11. Pharmazeutische Zusammensetzung nach Anspruch 1 oder 2 zur Verwendung bei der Behandlung der Alzheimer-Krankheit (AD).

## Revendications

1. Composition pharmaceutique, comprenant une composition présentant une activité anti-AChE, dans laquelle :
(a) la composition présentant une activité anti-AChE comprend de la columbamine, de la jatrorrhizine, de la palmatine et de la berbérine ;
(b) la composition pharmaceutique comprend en outre l'un quelconque ou une combinaison d'au moins deux parmi un support, excipient ou diluant pharmaceutiquement acceptable ; et
(i) la composition pharmaceutique est sous une forme liquide, et la columbamine, la jatrorrhizine et la palmatine dans la composition pharmaceutique présentent une concentration de 0,18 µg/mL, une concentration de 0,92 µg/mL, et une concentration de 0,63 µg/mL, respectivement, les composants de la composition présentent un meilleur effet synergique, FICI=0,49 ; ou
(ii) la composition pharmaceutique est sous une forme liquide, et la columbamine, la jatrorrhizine et la palmatine dans la composition pharmaceutique présentent une concentration de 0,93 µg/mL, une concentration de 0,92 µg/mL, et une concentration de 0,63 µg/mL, respectivement, les composants de la composition présentent un meilleur effet de superposition, FICI=0,99.

2. Composition pharmaceutique selon la revendication 1, dans laquelle dans la composition présentant une activité anti-AChE,
(a) un rapport massique de la columbamine, de la jatrorrhizine, de la palmatine et de la berbérine est de 1:5,16:9,24:10 ; ou
(b) un rapport massique de la columbamine, de la jatrorrhizine, de la palmatine et de la berbérine est de 0,9-1,1:4,644-5,676:8,316-10,164:9-11, et l'activité de la composition est de 90% à 110% d'activité d'une composition contenant de la columbamine, de la jatrorrhizine, de la palmatine et de la berbérine dont le rapport massique est de 1:5,16:9,24:10.

3. Procédé de criblage pour la composition présentant une activité anti-AChE dans la composition pharmaceutique selon la revendication 1 ou 2, comprenant les étapes suivantes :
(1) la préparation d'un réacteur : l'immobilisation de l'AChE sur une colonne monolithique de poly(GMA-*co*-EDMA) ;
(2) la réalisation d'une analyse de recherche de ligands : le passage d'un échantillon de Mahoniae Caulis à travers la colonne dans le réacteur à l'étape (1) et l'élution de l'échantillon de Mahoniae Caulis, et la collecte de fractions pour la LC-MS pour obtenir un ligand de liaison spécifique ; et
(3) l'identification d'une structure de ligand : la comparaison du ligand de liaison spécifique obtenu à l'étape (2) avec un standard au regard d'aspects relatifs au temps de rétention et aux données MS pour identifier la structure de ligand et obtenir la composition.

4. Procédé selon la revendication 3, dans lequel un procédé de préparation du réacteur à l'étape (1) comprend la modification d'un groupe alcényle sur une paroi interne d'un capillaire, la préparation de la colonne monolithique de poly(GMA-*co*-EDMA) avec du méthacrylate de glycidyle et du diméthylacrylate d'éthylène glycol comme monomères fonctionnels, et l'immobilisation de l'AChE sur la colonne monolithique de poly(GMA-*co*-EDMA).

5. Procédé selon la revendication 3 ou 4, dans lequel la chromatographie en phase liquide couplée à la spectrométrie de masse à l'étape (2) utilise de l'acétonitrile comme phase mobile A et utilise un mélange d'acide formique et d'acétate d'ammonium comme phase mobile B.

6. Procédé selon la revendication 5, dans lequel la phase mobile A et la phase mobile B présentent chacune un débit de 0,5-1,5 mL/min ;
de préférence, la LC-MS à l'étape (2) présente un volume d'injection de 2-4 µL, une température de colonne de 24-26°C, et une longueur d'onde de détection de 280 nm ;
de préférence, les conditions d'élution graduée de la LC-MS à l'étape (2) comprennent 0-17 min et 5%B-18%B ; 17-35 min et 18%B-21%B ; 35-45 min et 21%B ; 45-55 min et 21%B-25%B ; 55-65 min et 25%B-50%B ; ou 65-90 min et 50%B-70%B.

7. Procédé selon l'une quelconque des revendications 3 à 6, dans lequel le standard de l'étape (3) comprend un standard de columbamine, un standard de chlorhydrate de jatrorrhizine, un standard de chlorhydrate de palmatine et un standard de chlorhydrate de berbérine.

8. Procédé selon l'une quelconque des revendications 3 à 7, comprenant en outre, après l'étape (3), une étape de détermination de l'activité anti-AChE du ligand ;
de préférence, l'étape de détermination de l'activité anti-AChE du ligand comprend le mélange d'AChE avec le ligand, l'ajout d'acide dithiobis-nitrobenzoïque et d'ATCI, la mesure de l'absorbance après incubation, et le calcul d'un taux d'inhibition d'AChE.

9. Procédé selon la revendication 8, dans lequel l'AChE présente une concentration finale de 0,0301-0,0305 U/mL ;
de préférence, l'acide dithiobis-nitrobenzoïque présente une concentration finale de 0,32-0,34 mM ;
de préférence, l'ATCI présente une concentration finale de 1,4-1,6 mM ;
de préférence, l'incubation est effectuée à une température de 35-38°C ; et
de préférence, l'incubation est effectuée pendant 5-15 min.

10. Procédé selon l'une quelconque des revendications 3 à 9, comprenant les étapes suivantes :
(1) la préparation du réacteur : la modification d'un groupe alcényle sur une paroi interne d'un capillaire, la préparation de la colonne monolithique de poly(GMA-*co-*EDMA) avec du méthacrylate de glycidyle et du diméthylacrylate d'éthylène glycol comme monomères fonctionnels, et l'immobilisation de l'AChE sur la colonne monolithique de poly(GMA-*co*-EDMA) pour obtenir le réacteur ;
(2) la réalisation de l'analyse de recherche de ligands : le passage de l'échantillon de Mahoniae Caulis à travers la colonne dans le réacteur à l'étape (1) et l'élution de l'échantillon de Mahoniae Caulis, et la collecte des fractions pour la LC-MS pour obtenir le ligand de liaison spécifique, dans lequel la LC-MS utilise de l'acétonitrile comme phase mobile A et utilise un mélange d'acide formique et d'acétate d'ammonium comme phase mobile B, dans lequel la phase mobile A et la phase mobile B présentent chacune un débit de 0,5-1,5 mL/min ; la LC-MS présente un volume d'injection de 2-4 µL, une température de colonne de 24-26°C, et une longueur d'onde de détection de 280 nm ; et les conditions d'élution graduée de la LC-MS comprennent 0-17 min et 5%B-18%B ; 17-35 min et 18%B-21%B ; 35-45 min et 21%B ; 45-55 min et 21%B-25%B ; 55-65 min et 25%B-50%B ; ou 65-90 min et 50%B-70%B ;
(3) l'identification de la structure de ligand : la comparaison du ligand de liaison spécifique obtenu à l'étape (2) avec un standard de columbamine, un standard de chlorhydrate de jatrorrhizine, un standard de chlorhydrate de palmatine, et un standard de chlorhydrate de berbérine au regard d'aspects relatifs au temps de rétention et aux données de spectrométrie de masse pour identifier la structure de ligand ; et
(4) la détermination de l'activité du ligand : le mélange de l'AChE avec le ligand, l'ajout de l'acide dithiobis-nitrobenzoïque et de l'iodure d'acétylthiocholine, la mesure de l'absorbance après incubation pendant 5-15 min à 35-38°C, et le calcul d'un taux d'inhibition d'AChE.

11. Composition pharmaceutique selon la revendication 1 ou 2, pour son utilisation dans le traitement de la maladie d'Alzheimer (MA).
